# EUROPÄISCHE PATENTSCHRIFT

(11) **EP 3 585 185 B1**
(45) Veröffentlichungstag und Bekanntmachung des Hinweises auf die Patenterteilung: **10.04.2024**
(21) Anmeldenummer: 17708202.1
(22) Anmeldetag: 27.02.2017
(51) Int. Cl.: A23L 27/20, C07C 49/637, C07C 49/653, C07C 13/52, C07C 33/16, C11B 9/00

(54) **VERFAHREN ZUR HERSTELLUNG VON ROTUNDON-HALTIGEN MISCHUNGEN**
METHOD FOR PRODUCING ROTUNDONE-CONTAINING MIXTURES
PROCÉDÉ POUR PRODUIRE DES MÉLANGES CONTENANT DE LA ROTUNDONE

(43) Veröffentlichungstag der Anmeldung: 01.01.2020
(73) Patentinhaber: Symrise AG, 37603 Holzminden (DE)
(72) Erfinder: BACKES, Michael, 37603 Holzminden (DE); VÖSSING, Tobias, 37688 Beverungen (DE); HEINEMEIER, Nadine, 37639 Bevern (DE); MEIER, Lars, 37691 Derental (DE); SCHATKOWSKI, Dietmar, 37574 Einbeck (DE); KRIEGER, Willi, 33034 Brakel (DE); REICHELT, Katharina, 37603 Holzminden (DE); OTTE-HÖLSCHER, Susanne, 37620 Halle (DE)
(74) Vertreter: Eisenführ Speiser
(86) Internationale Anmeldenummer: PCT/EP2017/054524
(87) Internationale Veröffentlichungsnummer: WO 2018/153499

(56) Entgegenhaltungen:
- WO-A1-2012/001018
- CH-A- 263 793
- CLAUDIA WOOD ET AL: "From Wine to Pepper: Rotundone, an Obscure Sesquiterpene, Is a Potent Spicy Aroma Compound", JOURNAL OF AGRICULTURAL AND FOOD CHEMISTRY, Bd. 56, Nr. 10, 1. Mai 2008 (2008-05-01), Seiten 3738-3744, XP055366765, ISSN: 0021-8561, DOI: 10.1021/jf800183k in der Anmeldung erwähnt
- Loïc Tissandié ET AL: "Revisiting the Chemistry of Guaiacwood Oil: Identification and Formation Pathways of 5,11- and 10,11-Epoxyguaianes", Journal of natural products, vol. 80, no. 2, 24 February 2017 (2017-02-24), pages 526-537, XP055737548, US ISSN: 0163-3864, DOI: 10.1021/acs.jnatprod.6b01068

## Beschreibung

Die vorliegende Erfindung betrifft gemäß einem primären Aspekt ein Verfahren zur Herstellung einer Mischung umfassend Rotundon. Ein weiterer Aspekt der vorliegenden Erfindung betrifft Rotundon-haltige Mischungen sowie deren Verwendung zum Erzeugen, Vermitteln oder Modifizieren, vorzugsweise Verstärken, eines oder mehrerer Geschmacks- und/oder Geruchseindrücke. Die Erfindung betrifft zudem eine der Ernährung, der Mundpflege oder dem Genuss dienende oder kosmetische oder zur oralen Aufnahme bestimmte pharmazeutische Zubereitung oder Halbfertigware zur Herstellung einer der Ernährung, der Mundpflege oder dem Genuss dienenden oder kosmetischen oder zur oralen Aufnahme bestimmten pharmazeutischen Zubereitung, umfassend eine erfindungsgemäße Mischung sowie des Weiteren ein Verfahren zur Herstellung besagter Zubereitung oder Halbfertigware.

Rotundon ((3S,5R,8S)-3,4,5,6,7,8-Hexahydro-3,8-dimethyl-5-(1-methylethenyl)-1(2H)-azulenon, CAS# 18374-76-0) wurde in der Natur in verschiedenen Weinen sowie schwarzem Pfeffer und vielen Kräutern nachgewiesen (J. Agric. Food Chem. 2008, 56, 3738-3744). Des Weiteren wird Rotundon in Cypriol-Öl als Stoff mit dem deutlichsten Impact beschrieben *(*J. Agric. Food. Chem. 2016, 64, 4566-4573). Auch in Weihrauch kommt Rotundon vor (J. Nat. Prod. 2016, 79, 1160-1164).

In WO 2015 181 257 werden Parfümkompositionen mit besonderen raumzeitlichen Profilen beschrieben, die unter vielem anderen auch Rotundon enthalten können.

Rotundon wurde zudem in diversen Früchten (explizit genannt: Grapefruit, Orange, Apfel und Mango) identifiziert und die positive Auswirkung von Rotundon in Fruchtaromen sensorisch beschrieben, wobei hier eher die holzigen Noten im Vordergrund stehen (Poster auf dem Wartburg Symposium **2016**). Von der gleichen Gruppe wurde kürzlich in der Offenlegungsschrift JP 2016 198 026 die Verstärkung von Fruchtaromen beschrieben.

Die Herstellung von Rotundon aus Guajol (extrahiert aus Guajakholzöl) über das entsprechende Guajen ist ebenfalls in der Literatur beschrieben. Es werden bei der beschriebenen Synthese jedoch der Reinstoff benutzt und metallorganische Reagenzien eingesetzt (J. Agric. Food Chem. 2008, 56, 3738-3744 und Indian Journal of Chemistry, Section B: Organic Chemistry Including Medicinal Chemistry 1983, 22B, 1060).

Das Dokument WO 2011/106166 A1 beschreibt ebenfalls die Herstellung von Rotundon aus Guajen durch Oxidation unter Verwendung eines metallbasierten Katalysators.

EP 2 588 436 B1 offenbart die Herstellung von Rotundon direkt aus Guajen unter Verwendung von Laccase. Dabei entsteht eine Mischung aus Rotundon und dem entsprechenden Rotundol sowie weiteren Nebenkomponenten.

Ebenfalls beschrieben ist die Autoxidation von Guajen in organischen Lösungsmitteln bzw. auf Filterpapier (J. Agric. Food Chem. 2014, 62, 10809-10815). Zudem sind Studien über den Mechanismus der besagten Autoxidation bekannt (J. Nat. Prod. 2015, 78, 131-145; J. Agric. Food Chem. 2015, 63, 1932-1938).

Kürzlich wurde eine verbesserte Strategie für die Synthese von Rotundon mit Hilfe von Elektrochemie vorgestellt *(*Nature 2016, 533, 77-81). Allerdings ist bisher keine Synthesestrategie in der Literatur offenbart, mit deren Hilfe Rotundon in industriell relevantem Maßstab und akzeptabler Reinheit dargestellt werden kann.

Die Aromenindustrie ist ständig auf der Suche nach neuen Rohstoffen, die die Formulierung von authentischen, natürlichen Aromen ermöglichen. Rotundon werden diese Eigenschaften zugeschrieben, allerdings fehlte es bislang an einer industriell umsetzbaren Herstellungsmöglichkeit dieses Rohstoffes (insbesondere im Rahmen der relevanten EU-Gesetzgebung).

Überraschenderweise wurde im Rahmen der dieser Erfindung zugrunde liegenden Untersuchungen nun festgestellt, dass durch die Behandlung von Holzölen, Extrakten und/oder Naturharzen enthaltend Guajol und Bulnesol, insbesondere von Guajakholzöl, mit organischen Säuren und Luft (gegebenenfalls unter Zusatz eines Lösungsmittels) eine Mischung erhalten wird, die neben Rotundon noch weitere Komponenten enthält, die beim Einsatz in Aromen deren Authentizität und Impact vorteilhafterweise sogar noch steigern. Die erfindungsgemäßen Mischungen können vorteilhafterweise als natürliche Aromen gemäß Verordnung (EG) Nr. 1334/2008 des Europäischen Parlaments und des Rates über Aromen und bestimmte Lebensmittelzutaten mit Aromaeigenschaften zur Verwendung in und auf Lebensmitteln eingesetzt werden.

Die vorliegende Erfindung betrifft somit gemäß einem primären Aspekt ein Verfahren zur Herstellung einer Mischung umfassend Rotundon, umfassend oder bestehend aus folgenden Schritten:
a) Umsetzen von Holzöl(en), Extrakt(en) und/oder Naturharz(en), bevorzugt von Guajakholzöl, enthaltend Guajol und Bulnesol, bevorzugt enthaltend 15 bis 45 Gew.-% Guajol und 25 bis 55 Gew.-% Bulnesol, besonders bevorzugt enthaltend 20 bis 35 Gew.-% Guajol und 30 bis 45 Gew.-% Bulnesol, mit einer oder mehreren organischen Säure(n), 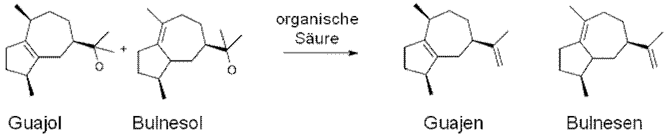
b) Abtrennen der Guajen-haltigen Fraktion von der in Schritt a) erhaltenen bzw. bereitgestellten Mischung, bevorzugt durch Destillation,
c) Oxidation der abgetrennten Guajen-haltigen Fraktion aus Schritt b), bevorzugt mit Umgebungsluft und/oder reinem Sauerstoff als Oxidationsmittel, 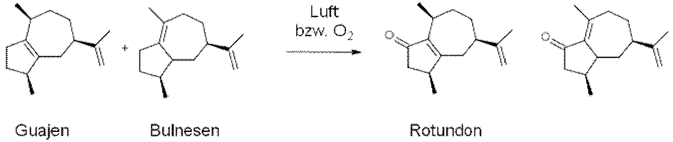
d) optional: Erhitzen der in Schritt c) erhaltenen oxidierten Mischung, bevorzugt Erhitzen über einen Zeitraum von 1 bis 3 h und/oder bei 110 bis 150 °C, vorzugsweise bei 115 bis 125 °C,
e) Abtrennen der Rotundon-haltigen Fraktion von der in Schritt c) oder d), falls vorhanden, erhaltenen Mischung, bevorzugt durch Destillation, um die Rotundon-haltige Mischung zu erhalten.

Bei dem/den in Schritt a) des erfindungsgemäßen Verfahrens eingesetzten Holzöl(en) handelt es sich bevorzugt um Guajakholzöl, vorzugsweise gewonnen aus dem Holz der Baumart *Bulnesia sarmientoi*, und/oder um Holzöl gewonnen aus der Art *Callitris glaucophylla.* Bei dem/den in Schritt a) des erfindungsgemäßen Verfahrens eingesetzten Naturharz(en) handelt es sich bevorzugt um Guajakharz, vorzugsweise gewonnen aus der Baumart *Bulnesia sarmientoi, Guaiacum officinale* und/oder *Guaiacum sanctum.* Es ist im Rahmen der vorliegenden Erfindung zudem möglich, Extrakte bestimmter Pflanzenarten, vorzugsweise von Ferula-Arten, als Quellen von Guajol und Bulnesol einzusetzen.

Guajakholzöl enthaltend Guajol und Bulnesol wird vorzugsweise durch Wasserdampfdestillation aus der Rinde oder dem Holz der Baumart *Bulnesia sarmientoi* extrahiert. Des Weiteren können auch andere Guajak-Spezies, z.B. *Guaiacum officinale* und/oder *Guaiacum sanctum* als Ausgangsstoff benutzt werden.

Bei dem in Schritt c) des erfindungsgemäßen Verfahrens eingesetzten reinen Sauerstoff, falls vorhanden, handelt es sich bevorzugt um Sauerstoff mit einer Reinheit von ≥ 99.9 Vol.-%, weiter bevorzugt von ≥ 99.99 Vol.-%, besonders bevorzugt von ≥ 99.998 Vol.-%. Vorzugsweise wird in Schritt c) des erfindungsgemäßen Verfahrens Umgebungsluft (d.h. der in der Umgebungsluft enthaltene Sauerstoff) als Oxidationsmittel eingesetzt. Dies ermöglicht ein besonders kostengünstiges Herstellungsverfahren von Mischungen enthaltend Rotundon, insbesondere in industriellem Maßstab.

Das erfindungsgemäße Verfahren ist besonders vorteilhaft, da dessen Produkte (d.h. die erfindungsgemäßen Mischungen umfassend Rotundon), Edukte sowie auch alle weiteren im Verfahren eingesetzten Stoffe lebensmitteltechnologisch und -rechtlich ohne Bedenken einsetzbar sind. Vorteilhafterweise beruht das Verfahren auf dem Einsatz nachwachsender Rohstoffe und verwendet keine metallorganischen Reagenzien. Das erfindungsgemäße Verfahren erfordert zudem keine komplexen oder ungewöhnlichen Verfahrensschritte oder Prozeduren, so dass es in herkömmlichen Produktionsanlagen problemlos und in industriellem Maßstab einsetzbar ist. Die mit dem erfindungsgemäßen Verfahren erhaltene Mischung umfassend Rotundon ist besonders stabil und somit in Aromen und Lebensmitteln breit einsetzbar und mit vielen anderen Komponenten kompatibel.

Gemäß einer bevorzugten Ausführungsform des erfindungsgemäßen Verfahrens ist bzw. sind die in Schritt a) eingesetzte(n) organische(n) Säure(n) ausgewählt aus der Gruppe bestehend aus Citronensäure, Äpfelsäure, Weinsäure und Oxalsäure, vorzugsweise ist die in Schritt a) eingesetzte organische Säure Weinsäure.

Dabei kann die organische Säure gemäß einer Ausführungsform komplett am Anfang der Umsetzung in Schritt a) des Verfahrens zugegeben werden. Als bevorzugt hat sich jedoch eine wiederholte Zugabe kleinerer Mengen erwiesen. Vorzugsweise werden daher am Anfang der Umsetzung in Schritt a) des Verfahrens die Hälfte der Säure zugegeben und nach der Hälfte der Reaktionszeit der Umsetzung in Schritt a) die zweite Hälfte der Säure zugegeben.

Gemäß einer weiteren bevorzugten Ausführungsform des erfindungsgemäßen Verfahrens liegt in Schritt a) das Gewichtsverhältnis von Holzöl(en), Extrakt(en) und/oder Naturharz(en) enthaltend Guajol und Bulnesol zu organischer Säure bzw. zu organischen Säuren zwischen 50:1 und 3:1, bevorzugt zwischen 20:1 und 2:1, besonders bevorzugt zwischen 12:1 und 8:1.

Gemäß einer weiteren bevorzugten Ausführungsform des erfindungsgemäßen Verfahrens findet die Umsetzung in Schritt a) in Gegenwart eines Co-Solvenzes oder mehrerer Co-Solvenzien, bevorzugt in Gegenwart eines Polyethylenglycols oder mehrerer Polyethylenglycole, besonders bevorzugt in Gegenwart von PEG 600, statt und vorzugsweise beträgt das Gewichtsverhältnis von Holzöl(en), Extrakt(en) und/oder Naturharz(en) enthaltend Guajol und Bulnesol zu dem einen oder den mehreren Co-Solvenz(ien) 4:1 bis 1:2.

Die Zugabe eines Co-Solvenzes oder mehrerer Co-Solvenzien zu der Mischung vor oder während der Umsetzung in Schritt a) des Verfahrens ist dabei besonders vorteilhaft, da die Anwesenheit eines Co-Solvenzes oder mehrerer Co-Solvenzien die Rührbarkeit der Reaktion sicherstellt.

Gemäß einer weiteren bevorzugten Ausführungsform des erfindungsgemäßen Verfahrens liegt die Reaktionstemperatur der Umsetzung in Schritt a) zwischen 160 und 260 °C, bevorzugt zwischen 180 und 220 °C, und/oder die Reaktionszeit der Umsetzung in Schritt a) zwischen 1 und 20 h, bevorzugt zwischen 5 und 15 h, und/oder erfolgt die Umsetzung in Schritt a) bei Normaldruck oder bei Unterdruck, vorzugsweise bei 400 bis 900 mbar, besonders bevorzugt bei 500 bis 700 mbar.

Unter Normaldruck ist im Rahmen der vorliegenden Erfindung der Umgebungsdruck ohne das Anlegen eines Vakuums zu verstehen. Dieser beträgt auf Meereshöhe üblicherweise circa 1013,25 hPa bzw. 1.01325 bar und bei höheren Lagen entsprechend weniger.

Die in Schritt a) des erfindungsgemäßen Verfahrens ablaufende Umsetzung bzw. Dehydratisierungsreaktion erfolgt vorzugsweise bei einem Unterdruck wie vorangehend definiert, um die Entfernung des entstehenden Reaktionswassers zu verbessern bzw. zu vereinfachen.

Gemäß einer weiteren bevorzugten Ausführungsform des erfindungsgemäßen Verfahrens erfolgt in Schritt b) das Abtrennen der Guajen-haltigen Fraktion von der in Schritt a) erhaltenen Mischung durch Destillation, bevorzugt bei Unterdruck, vorzugsweise bei 1,0 bis 20,0 mbar und einer Sumpftemperatur von 90 bis 175 °C, besonders bevorzugt bei 2,0 bis 10,0 mbar und einer Sumpftemperatur von 105 bis 160 °C, weiter bevorzugt bei 4,0 mbar und einer Sumpftemperatur von 130 °C.

Vorzugsweise beträgt der Anteil an Guajen in der in Schritt b) gewonnen Guajen-haltigen Fraktion 18 Gew.-% oder mehr.

Gemäß einer weiteren bevorzugten Ausführungsform des erfindungsgemäßen Verfahrens findet in Schritt c) die Oxidation der Guajen-haltigen Fraktion aus Schritt b) mit Umgebungsluft und/oder reinem Sauerstoff als Oxidationsmittel und/oder bei einer Temperatur von 60 bis 150 °C, bevorzugt 80 bis 130 °C, besonders bevorzugt 90 bis 110°C, und/oder mit einer Gasmenge des Oxidationsmittels von ≥ 10 bis ≤ 1000 l/h, bevorzugt von ≥ 30 bis ≤ 500 l/h, besonders bevorzugt von ≥ 40 bis ≤ 100 l/h, pro kg Edukt und/oder über einen Zeitraum von 10 bis 60 h, bevorzugt von 15 bis 30 h, statt.

Unter Edukt ist im Rahmen dieser bevorzugten Ausführungsform die in Schritt b) des erfindungsgemäßen Verfahrens gewonnene Guajen-haltige Fraktion zu verstehen.

Der Einsatz der vorangehend definierten Gasmengen des Oxidationsmittels in Schritt c) des Verfahrens ist besonders vorteilhaft, da bei geringeren Luftmengen oftmals die Ausbeute zu gering ist und höhere Luftmengen zur Bildung von Nebenprodukten oder Zersetzung der entstehenden gewünschten Produkte führen.

Vorzugsweise wird die Oxidation in Schritt c) des erfindungsgemäßen Verfahrens so lange durchgeführt, bis eine merkliche Abnahme der Reaktionsgeschwindigkeit einsetzt. Die Abnahme der Reaktionsgeschwindigkeit wird vorzugsweise durch Probenentnahme (aus dem Reaktionskolben) und Analyse oder durch Messung des Sauerstoffverbrauchs in der Abluft während der Oxidationsreaktion bestimmt. Vorzugsweise liegt das Gewichtsverhältnis von nicht umgesetztem Guajen zu Rotundon nach Beendigung der Oxidationsreaktion in Schritt c) zwischen 12:1 und 3:1 und liegt der Gehalt an Rotundon in dieser Mischung bei > 2000 ppm, besonders bevorzugt bei > 3500 ppm, idealerweise bei > 6000 ppm.

Gemäß einer weiteren bevorzugten Ausführungsform des erfindungsgemäßen Verfahrens beträgt die Peroxidzahl der in Schritt c) oder d), falls vorhanden, erhaltenen Mischung < 30 meq O/kg, bevorzugt < 20 meq O/kg beträgt.

Gemäß einer bevorzugten Ausführungsform ist Schritt d) im erfindungsgemäßen Verfahren vorhanden, um vorteilhafterweise eventuell entstandene Peroxide zu zerstören. Bei Wahl der besonders geeigneten Bedingungen in Schritt d) des Verfahrens wie vorangehend definiert, bleibt der Gehalt an Rotundon in der Mischung weitestgehend erhalten. Bei höheren Temperaturen läuft die Zersetzung der Peroxide in Schritt d) schneller ab, allerdings nimmt dadurch der Gehalt an Rotundon erheblich ab.

Die erhaltenen Werte des Rotundon-Gehalts in der Mischung nach Schritt c) bzw. nach Schritt d), falls vorhanden, des erfindungsgemäßen Verfahrens wurden semiquantitativ (gegen 2-Nonanol und/oder Tridecan als internen Standard), vorzugsweise mit Hilfe gaschromatographischer Methoden, bestimmt. Je nach Methode und gewähltem Standard können diese Werte abweichen.

Gemäß einer weiteren bevorzugten Ausführungsform des erfindungsgemäßen Verfahrens wird der Mischung in Schritt e) vor und/oder während der Destillation ein oder mehr Schleppmittel ausgewählt aus der Gruppe bestehend aus Triacetin und Triethylcitrat im Gewichtsverhältnis von in Schritt c) oder d), falls vorhanden, erhaltener Mischung zu Schleppmittel von 4:1 bis 1:4, bevorzugt von 3:1 bis 1:1,
und/oder
ein oder mehr Co-Solvenz(ien) ausgewählt aus der Gruppe bestehend aus Polyethylenglycolen und Palatinol Z (CAS# 68515-9-1), bevorzugt PEG 400 und/oder PEG 600, im Gewichtsverhältnis von in Schritt c) oder d), falls vorhanden, erhaltener Mischung zu Co-Solvenz(ien) von 10:1 bis 1:5, bevorzugt von 5:1 bis 1:1,
zugegeben.

Die Zugabe eines oder mehrerer Schleppmittel(s) und/oder Co-Solvenz(ien) vor und/oder während der Destillation in Schritt e) des erfindungsgemäßen Verfahrens ist besonders vorteilhaft, da das/die Schleppmittel zu einer leichteren und effizienteren Destillation bzw. Abtrennung der Rotundon-haltigen Mischung führt/führen und die Zugabe von Co-Solvenz(ien) die Rührbarkeit des Destillationssumpfes sicherstellt. Vorzugsweise wird ein Schleppmittel verwendet, das als Lösungsmittel für Aromastoffe zugelassen ist.

Gemäß einer weiteren bevorzugten Ausführungsform des erfindungsgemäßen Verfahrens findet in Schritt e) das Abtrennen der Rotundon-haltigen Fraktion von der in Schritt c) oder d), falls vorhanden, erhaltenen Mischung durch Destillation, bevorzugt bei Unterdruck, vorzugsweise bei 3 bis 5 mbar, statt.

Vorzugsweise wird bei der Destillation in Schritt e) die in Schritt c) oder d), falls vorhanden, erhaltene Rotundon-haltige Fraktion von den nicht-flüchtigen polymeren Bestandteilen abgetrennt. Besonders bevorzugt wird eine Fraktion mit leichtsiedenden Komponenten (Kopftemperatur zwischen 95°C und 105°C bei 3 - 5 mbar), die kein bzw. sehr geringe Mengen an Rotundon enthält, vorher abgetrennt. Die Destillation ist dann beendet, wenn eine Kopftemperatur von 130 °C bis 160 °C bei 3 - 5 mbar erreicht wird.

Vorzugsweise wird das in Schritt e) erhaltene Destillat durch Zugabe von weiteren Trägerstoffen (vorzugsweise z.B. von vor und/oder während der Destillation in Schritt e) zugegebenem Schleppmittel, bevorzugt von Triethylcitrat) so standardisiert, dass in Schritt e) eine Mischung enthaltend 0,25 bis 2,50 Gew.-%, besonders bevorzugt enthaltend 0,50 bis 1,50 Gew.-%, Rotundon erhalten wird, welche dann direkt in Aromen eingearbeitet werden kann.

Gemäß einer weiteren Ausführungsform kann die in Schritt e) des erfindungsgemäßen Verfahrens erhaltene Rotundon-haltige Mischung erneut (fein-)destilliert werden, vorzugsweise so dass eine Mischung mit einem Rotundon-Gehalt von 3 bis 15 Gew.-%, vorzugsweise von 5 bis 10 Gew.-%, erhalten wird, wobei das Gewichtsverhältnis von Rotundon zu Triethylcitrat, falls vorhanden, in der nach der weiteren Destillation erhaltenen Mischung bevorzugt zwischen 1:3 und 1:10 liegt.

Die erhaltenen Werte für den Gehalt an Rotundon in der in Schritt e) des erfindungsgemäßen Verfahrens erhaltenen Rotundon-haltigen Fraktion wurden semiquantitativ (gegen 2-Nonanol oder Tridecan als internen Standard) bestimmt. Je nach Methode und gewähltem Standard können diese Werte abweichen. Für die Angaben wurde für die Messungen gegen Tridecan als internen Standard für Triethylcitrat ein Korrekturfaktor von 2,555 angesetzt.

Die in Schritt e) des erfindungsgemäßen Verfahrens erhaltene Rotundon-haltige Mischung, vorzugsweise enthaltend 0,25 bis 2,50 Gew.-%, besonders bevorzugt enthaltend 0,50 bis 1,50 Gew.-% Rotundon, wird vorzugsweise in einer Konzentration von 0,1 ppm bis 5000 ppm, besonders bevorzugt von 0,5 ppm bis 500 ppm, insbesondere bevorzugt von 1 ppm bis 200 ppm in Geschmacks- und/oder Geruchsstoffmischungen eingesetzt, und/oder vorzugsweise in einer Konzentration der Rotundon-haltigen Mischung im fertigen (ggf. die Geschmacks- und/oder Geruchsstoffmischung enthaltenden) Produkt (Halbfertigware oder Zubereitung) von vorzugsweise 0,0001 ppm bis 10,0 ppm, bevorzugt von 0,0001 ppm bis 1,0 ppm, besonders bevorzugt von 0,0005 ppm bis 0,5 ppm, insbesondere bevorzugt von 0,001 ppm bis 0,25 ppm.

Eine erfindungsgemäße Mischung oder eine Geschmacks- und/oder Geruchsstoffmischung enthaltend die in Schritt e) des erfindungsgemäßen Verfahrens erhaltene Rotundon-haltige Mischung können vor dem nachfolgenden Einsatz in Halbfertigwaren oder Zubereitungen, insbesondere solchen wie hierin beschrieben, in eine gewünschte Formulierung (z.B. durch Sprühtrocknung, vorzugsweise Evogran^{®}) überführt werden.

Eine solche (vorzugsweise sprühgetrocknete) Formulierung umfasst neben der in Schritt e) des erfindungsgemäßen Verfahrens erhaltenen Rotundon-haltigen Mischung vorzugsweise weitere Geruchs- und/oder Geschmacksstoffe sowie einen oder mehrere zum Verzehr geeignete feste Trägerstoffe.

Vorteilhafte Trägerstoffe in diesen bevorzugten erfindungsgemäßen (vorzugsweise sprühgetrockneten) Formulierungen sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine. Bevorzugte Trägerstoffe sind Siliciumdioxid, Gummi Arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Formulierungen, welche die in Schritt e) des erfindungsgemäßen Verfahrens erhaltene Rotundon-haltige Mischung enthalten, sowie zudem noch einen oder mehrere feste Trägerstoffe umfassen, können beispielsweise durch mechanische Mischvorgänge, wobei gleichzeitig auch eine Zerkleinerung der Partikel erfolgen kann, oder mittels Sprühtrocknung hergestellt werden. Bevorzugt sind erfindungsgemäße Formulierungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäße, Trägerstoffe umfassende Formulierungen, die mittels Sprühtrocknung hergestellt wurden, besitzen vorzugsweise eine mittlere Partikelgröße im Bereich von 30 - 300 µm und eine Restfeuchte von kleiner oder gleich 5 Gew.-%.

Ein weiterer Aspekt der vorliegenden Erfindung betrifft eine Rotundon-haltige Mischung, (a) umfassend oder bestehend aus Rotundon, Bulnesen, Guajen, Guajol und Bulnesol sowie optional Triethylcitrat und/oder Triacetin.

Für die erfindungsgemäße Rotundon-haltige Mischung, deren Herstellungsverfahren und dessen bevorzugte Ausführungsformen gilt das im Rahmen des erfindungsgemäßen Verfahrens vorangehend Gesagte entsprechend.

Bevorzugt ist eine erfindungsgemäße Rotundon-haltige Mischung enthaltend Triethylcitrat sowie die gesamten Bestandteile Rotundon, Bulnesen, Guajen, Bulnesol und Guajol in einem Gewichtsverhältnis von 1:1 bis 30:1, besonders bevorzugt von 2:1 bis 25:1, weiter bevorzugt von 5:1 bis 20:1. Vorzugsweise liegt das Gewichtsverhältnis von Rotundon zu der Gesamtmenge an Bulnesen und Guajen in der erfindungsgemäßen Rotundon-haltigen Mischung bei 20:1 bis 1:40, besonders bevorzugt bei 5:1 bis 1:20, weiter bevorzugt bei 2:1 bis 1:12. Vorzugsweise liegt des Weiteren in der erfindungsgemäßen Rotundon-haltigen Mischung das Gewichtsverhältnis von Rotundon zu der Gesamtmenge an Bulnesol und Guajol bei 25:1 bis 1:15, besonders bevorzugt bei 10:1 bis 1:10, insbesondere bevorzugt bei 5:1 bis 1:5.

Ein weiterer Aspekt der vorliegenden Erfindung betrifft die Verwendung einer erfindungsgemäßen Mischung wie vorangehend definiert zum Erzeugen, Vermitteln oder Modifizieren, vorzugsweise Verstärken, eines oder mehrerer Geschmacks- und/oder Geruchseindrücke, vorzugsweise ausgewählt aus der Gruppe bestehend aus Citrus-, Frucht-, Mint-, Muskatnuss-, Pfeffer-, Gewürz-, Kräuter-, Kaffee-, Schokolade, Nuss-, Nougat-Nuss-Eindrücken, gelagerte Spirituosen (z.B. Whiskey-, Rum-, Brandy-Eindrücken), Wein- und Savory-Eindrücken.

Gemäß einer bevorzugten Ausführungsform wird die erfindungsgemäße Mischung wie vorangehend definiert dazu verwendet, um anderen Geschmacksstoffen einen komplexeren, natürlichen (und gegebenenfalls verstärkten) Geschmackseindruck zu verleihen.

Ein weiterer Aspekt der vorliegenden Erfindung betrifft eine der Ernährung, der Mundpflege oder dem Genuss dienende oder kosmetische oder zur oralen Aufnahme bestimmte pharmazeutische Zubereitung oder Halbfertigware zur Herstellung einer der Ernährung, der Mundpflege oder dem Genuss dienenden oder kosmetischen oder zur oralen Aufnahme bestimmten pharmazeutischen Zubereitung, umfassend eine erfindungsgemäße Rotundon-haltige Mischung wie vorangehend definiert.

Der Ernährung oder dem Genuss dienende Zubereitungen im Sinne der vorliegenden Erfindung sind z.B. Backwaren (z.B. Brot, Trockenkekse, Kuchen, sonstiges Gebäck), Süßwaren (z.B. Schokoladen, Schokoladenriegelprodukte, sonstige Riegelprodukte, Fruchtgummi, Hart- und Weichkaramellen, Kaugummi), alkoholische oder nichtalkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die Zubereitungen im Sinne der Erfindung können auch in Form von Kapseln, Tabletten (nichtüberzogene sowie überzogene Tabletten, z.B. mit magensaftresistentem Überzug), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Zur oralen Aufnahme bestimmte pharmazeutische Zubereitungen im Sinne der vorliegenden Erfindung sind Zubereitungen, die z.B. in Form von Kapseln, Tabletten (nichtüberzogene sowie überzogene Tabletten, z.B. mit magensaftresistentem Überzug), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Der Mundpflege dienende Zubereitungen im Sinne der vorliegenden Erfindung sind insbesondere Mund- und/oder Zahnpflegemittel wie Zahnpasten, Zahngele, Zahnpulver, Mundwässer, Kaugummis und andere Mundpflegemittel.

Kosmetische Zubereitungen sind im Rahmen der vorliegenden Erfindung beispielsweise kosmetische Zubereitungen zur Applikation im Bereich des Körpers oder des Kopfes wie Seifen, andere Reinigungs- oder Pflegemittel für den Gesichtsbereich oder den Körper, Gesichtscremes, -lotionen oder -salben, Sonnenschutzmittel, Bartreinigungs- oder - pflegemittel, Rasierschäume, -seifen oder -gele, Lippenstifte oder andere Lippenkosmetika oder Lippenpflegemittel.

Bevorzugt in erfindungsgemäßen Halbfertigwaren und Zubereitungen enthaltene Mengen an Rotundon bzw. erfindungsgemäßer Mischung ergeben sich aus den Ausführungen weiter oben.

In einer erfindungsgemäßen Halbfertigware oder Zubereitung können zudem übliche Wirk-, Grund-, Hilfs- und Zusatzstoffe für die Ernährung, der Mundpflege oder dem Genuss dienende oder orale pharmazeutische Zubereitungen (d.h. zur oralen Anwendung bestimmte pharmazeutische Zubereitungen) oder kosmetische Zubereitungen in Mengen von 0,9 bis 99,999999 Gew.-%, vorzugsweise 10 bis 80 Gew.-%, bezogen auf das Gesamtgewicht der Halbfertigware oder Zubereitung, enthalten sein. Insbesondere kann eine erfindungsgemäße Halbfertigware oder Zubereitung Wasser in einer Menge bis zu 99,999999 Gew.-%, vorzugsweise 5 bis 80 Gew.-%, bezogen auf das Gesamtgewicht der Halbfertigware oder Zubereitung, enthalten.

Als weitere Bestandteile für erfindungsgemäße, der Ernährung oder dem Genuss dienende Zubereitungen oder Halbfertigwaren können übliche Grund-, Hilfs- und Zusatzstoffe für Nahrungs- oder Genussmittel in einer erfindungsgemäßen Zubereitung enthalten sein (wie oben beschrieben) oder für die Herstellung solcher Zubereitungen verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, weitere Geschmackskorrigenzien bzw. Geschmacksmodulatoren für unangenehme Geschmackseindrücke oder nicht unangenehme Geschmackseindrücke, insbesondere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), Bitterstoffe (z.B. Chinin, Coffein, Limonin, Amarogentin, Humolone, Lupolone, Catechine, Tannine), Süßstoffe (z.B. Saccharin, Cyclamat, Aspartam, Neotam), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, (ggf. weitere) natürliche oder naturidentische Aromastoffe oder Riechstoffe sowie Geruchskorrigentien.

Zahnpflegemittel (als Beispiele für erfindungsgemäße der Mundpflege dienende Zubereitungen), umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Süßstoffe, wie z.B. Saccharin, Geschmackskorrigenzien für unangenehme Geschmackseindrücke oder in der Regel nicht unangenehme Geschmackseindrücke, (ggf. weitere) geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, (ggf. weitere) Aromen und/oder Natriumbicarbonat oder Geruchskorrigentien.

Kaugummis (als weiteres Beispiel für erfindungsgemäße der Mundpflege dienende Zubereitungen), umfassen im allgemeinen eine Kaugummibase, d.h. eine beim Kauen plastisch werdende Kaumasse, Zucker verschiedener Arten, Zuckeraustauschstoffe, Süßstoffe, Zuckeralkoholen, Geschmackskorrigenzien bzw. Geschmacksmodulatoren für unangenehme oder in der Regel nicht unangenehme Geschmackseindrücke, (ggf. weitere) geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, (ggf. weitere) Aromen und Stabilisatoren oder Geruchskorrigentien.

Gemäß einer bevorzugten Ausführungsform der erfindungsgemäßen Zubereitung oder Halbfertigware wie vorangehend definiert, umfasst die erfindungsgemäße Zubereitung oder Halbfertigware zudem einen oder mehrere (weitere) Geschmacks- und/oder Geruchsstoffe, wobei die Gesamtmenge an erfindungsgemäßer Rotundon-haltiger Mischung wie vorangehend definiert ausreicht, um einen oder mehrere Geschmacks- und/oder Geruchseindrücke des/der (weiteren) Geschmacks- und/oder Geruchsstoffe zu modifizieren, vorzugsweise zu verstärken.

Bevorzugte zu modifizierende, vorzugsweise zu verstärkende, weitere Geschmacks- und/oder Geruchsstoffe im Sinne dieser Erfindung kommen insbesondere vor in folgenden Extrakten, etherischen Ölen, Concretes, Absolues, Resinen, Resinoiden, Balsamen bzw. Tinkturen: Amyrisöl; Angelicasamenöl; Angelicawurzelöl; Anisöl; Baldrianöl; Basilikumöl; Baummoos-Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreum-Absolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eichenmoos-Absolue; Elemiöl; Estragonöl; Eucalyptus-citriodora-Öl; Eucalyptusöl; Fenchelöl; Fichtennadelöl; Galbanumöl; Galbanumresin; Geraniumöl; Grapefruitöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl; Lavendel-Absolue; Lavendelöl; Lemongrasöl; Liebstocköl; Limettenöl destilliert; Limettenöl gepresst; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Selleriesamenöl; Spiklavendelöl; Sternanisöl; Styraxöl; Tagetesöl; Tannennadelöl; Teebaumöl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl.

Beispiele erfindungsgemäß einzusetzender weiterer Geschmacks- und/oder Geruchsstoffe sind zudem aufgelistet in der EU-Positivliste der Durchführungsverordnung (EU) Nr. 872/2012, die seit dem 22. April 2013 gültig ist. In dieser Liste hat die Europäische Kommission eine Liste von Aromastoffen veröffentlicht, die in der EU in der Lebensmittelindustrie verwendet werden dürfen. Weitere Beispiele sind zu finden in der Flavor Ingredient Library der amerikanischen FEMA (Flavor & Extract Manufacturers Association).

Die im Rahmen der vorliegenden Erfindung bevorzugt zu modifizierenden, vorzugsweise zu verstärkenden, weiteren Geschmacks- und/oder Geruchsstoffe gehören unterschiedlichen chemischen Gruppen an, wie beispielsweise:
der Gruppe der Kohlenwasserstoffe, wie z.B. 3-Caren; alpha-Pinen; beta-Pinen; alpha-Terpinen; gamma-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien;
der Gruppe der aliphatischen Alkohole wie z.B. Hexanol; Octanol; 3-Octanol; 2,6-Dimethyl-heptanol; 2-Methylheptanol, 2-Methyloctanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7 Dimethyl-7-methoxyoctan-2-ol; 9-Decenol; 10-Undecenol; 4-Methyl-3-decen-5-ol;
der Gruppe der aliphatischen Aldehyde und deren Acetale wie z. B. Hexanal; Heptanal; Octanal; Nonanal; Decanal; Undecanal; Dodecanal; Tridecanal; 2-Methyloctanal; 2 Methylnonanal; (E)-2-Hexenal; (Z)-4-Heptenal; 2,6-Dimethyl-5-heptenal; 10 Undecenal; (E)-4-Decenal; 2-Dodecenal; 2,6,10-Trimethyl-5,9-undecadienal; Heptanaldiethylacetal; 1,1-Dimethoxy-2,2,5-trimethyl-4-hexen;
der Gruppe der aliphatischen Ketone und deren Oxime wie z.B. 2-Heptanon; 2-Octanon; 3-Octanon; 2-Nonanon; 5-Methyl-3-heptanon; 5-Methyl-3-heptanonoxim; 2,4,4,7-Tetramethyl-6-octen-3-on; der aliphatischen schwefelhaltigen Verbindungen wie z.B. 3-Methylthiohexanol; 3-Methylthiohexylacetat; 3-Mercaptohexanol; 3-Mercaptohexylacetat; 3-Mercaptohexylbutyrat; 3-Acetylthiohexylacetat; 1 Menthen-8-thiol;
der Gruppe der aliphatischen Nitrile wie z.B. 2-Nonensäurenitril; 2-Tridecensäurenitril; 2,12-Tridecadiensäurenitril; 3,7-Dimethyl-2,6-octadiensäurenitril; 3,7-Dimethyl-6-octensäu renitril;
der Gruppe der aliphatischen Carbonsäureester wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat; Isoamylbutyrat; Hexylbutyrat; (E)- und (Z)-3-Hexenylisobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat; Menthyllactat (FEMA GRAS 3748); Monomethylsuccinat (FEMA GRAS 3810); Monomenthylglutarat (FEMA GRAS 4006);
der Gruppe der acyclischen Terpenalkohole wie z.B. Citronellol; Geraniol; Nerol; Linalool; Lavadulol; Nerolidol; Farnesol; Tetrahydrolinalool; Tetrahydrogeraniol; 2,6 Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate, 3-Methyl-2-butenoate;
der Gruppe der acyclischen Terpenaldehyde und -ketone wie z.B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der Gruppe der cyclischen Terpenalkohole wie z.B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; sowie deren Formiate, Acetate, Propionate, Isobutyrate, Butyrate, Isovalerianate, Pentanoate, Hexanoate, Crotonate, Tiglinate, 3-Methyl-2-butenoate;
der Gruppe der cyclischen Terpenaldehyde und -ketone wie z.B. Menthon; Isomenthon; 8-Mercaptomenthan-3-on; Carvon; Campher; Fenchon; alpha-lonon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on; Nootkaton; Dihydronootkaton; alpha-Sinensal; beta-Sinensal;
der Gruppe der cyclischen Alkohole wie z.B. 4-tert.-Butylcyclohexanol; 3,3,5-Trimethylcyclo-hexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der Gruppe der cycloaliphatischen Alkohole wie z.B. alpha,3,3-Trimethylcyclohexylmethanol; 2 Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der Gruppe der Ester cycloaliphatischer Carbonsäuren wie z.B. Allyl-3-cyclohexylpropionat; Allylcyclohexyloxyacetat; Methyldihydrojasmonat; Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der Gruppe der aromatischen Kohlenwasserstoffe wie z.B. Styrol und Diphenylmethan;
der Gruppe der araliphatischen Alkohole wie z.B. Benzylalkohol; 1-Phenylethylalkohol; 2 Phenylethylalkohol; 3-Phenylpropanol; 2-Phenylpropanol; 2-Phenoxyethanol; 2,2 Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Gruppe der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen und aliphatischen Ether und Acetale bzw. Ketale wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; Menthyl Methyl Ether; Menthone Glyceryl Acetal (FEMA GRAS 3807); Menthone Glyceryl Ketal (FEMA GRAS 3808); Menthoxy-1,2-propandiol (FEMA GRAS 3784); Menthoxy-2-methyl-1,2-propandiol (FEMA GRAS 3849):
   der Gruppe der Carbonsäureamide wie z.B. WS-3 (FEMA GRAS 3455), WS-5 (FEMA GRAS 4309), WS-12 (Frescolat^{®} SC-1, FEMA GRAS 4681), WS-23 (FEMA GRAS 3804), (E)-3-Benzo[1,3]Dioxol-5-yl-N,N-Diphenyl-2-Propenamid (FEMA GRAS 4788) und 2-(4-Methylphenoxy)-N-(1H-pyrazol-3-yl)-N-(thiophen-2-ylmethyl)acetamid (FEMA GRAS 4809); N-(2-isopropyl-5-methylcyclohexyl)cyclopropancarboxamid (FEMA GRAS 4558), 3-(3,4-Dimethoxyphenyl)-N-[2-(3,4-dimethoxyphenyl)-ethyl]-acrylamid (FEMA GRAS 4310); (E)-N-[2-(1,3-Benzodioxol-5-yl)ethyl]-3-(3,4-dimethoxyphenyl)prop-2-enamid (FEMA GRAS 4773);
   der Gruppe der Carbonate und Harnstoffe wie z.B. Menthol Ethylen Glycol Carbonat (FEMA GRAS 3805); Menthol Propylenglycol Carbonat (FEMA GRAS 3806); 3-[3-(2-Isopropyl-5-methyl-cyclohexyl)ureido]buttersäureethylester(FEMA GRAS 4766);
   der Gruppe der aromatischen und araliphatischen Aldehyde wie z.B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4 Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
   der Gruppe der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon; Benzophenon;
   der Gruppe der aromatischen und araliphatischen Carbonsäuren und deren Ester wie z.B. Benzoesäure; Phenylessigsäure; Methylbenzoat; Ethylbenzoat; Hexylbenzoat; Benzylbenzoat; Methylphenylacetat; Ethylphenylacetat; Geranylphenylacetat; Phenylethyl-phenylacetat; Methylcinnamat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
   der Gruppe der stickstoffhaltigen aromatischen Verbindungen wie z.B. 2,4,6-Trinitro-1,3-dime-thyl-5-tert.-butylbenzol; 3,5-Dinitro-2,6-dimethyl-4-tert.-butylacetophenon; Zimtsäurenitril; 5-Phenyl-3-methyl-2-pentensäurenitril; 5-Phenyl-3-methylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexen-carbaldehyd; 6-Isopropylchinolin; 6-Isobutylchinolin; 6-sec.-Butylchinolin; Indol; Skatol; 2-Methoxy-3-isopropylpyrazin; 2-Isobutyl-3-methoxypyrazin;
   der Gruppe der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2 Ethoxy-5-(1-propenyl)phenol; p-Kresylphenylacetat;
   der Gruppe der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2 Ethyl-3-hydroxy-4H-pyran-4-on;
   der Gruppe der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Gemäß einem weiteren Aspekt betrifft die vorliegende Erfindung ein Verfahren zur Herstellung einer erfindungsgemäßen Zubereitung oder Halbfertigware wie vorangehend definiert, umfassend oder bestehend aus folgenden Schritten:
- Bereitstellen einer Rotundon-haltigen Mischung wie vorangehend definiert sowie eines oder mehrerer weiterer Bestandteile, wobei der weitere Bestandteil bzw. ein weiterer Bestandteil oder mehrere weitere Bestandteile vorzugsweise ausgewählt ist / sind aus der Gruppe bestehend aus Geschmacks- und/oder Geruchsstoffen, und
- Mischen der Rotundon-haltigen Mischung wie vorangehend definiert mit dem bzw. den weiteren Bestandteilen.

Nachfolgend wird die Erfindung anhand von Beispielen näher erläutert. Sofern nicht anders angegeben, beziehen sich alle Angaben, insbesondere Prozent- und Mengenangaben, auf das Gewicht. Die Mengenangabe der Einsatzstoffe in den Anwendungsbeispielen erfolgt - wenn nicht explizit für das jeweilige Beispiel anders beschrieben - in [g]. Der Begriff "Q.S." steht für *quantum satis.*

### Beispiele

### Herstellung erfindungsgemäßer Rotundon-haltiger Mischungen

### Beispiel 1a: Dehydratisierung von Guajakholzöl mit Weinsäure

Es werden 1000 g Guajakholzöl, 50 g Weinsäure und 500 g PEG 600 vorgelegt und auf 210°C aufgeheizt. Ab 180°C entsteht Reaktionswasser, welches kontinuierlich abdestilliert wird. Nach 2 h werden nochmals 50 g Weinsäure nachgegeben und für weitere 1,5 h bei 210°C gerührt. Durch Destillation des Reaktionsgemisches bei einer Sumpftemperatur von 130°C und einem Vakuum von 4,0 mbar werden 766 g einer Fraktion mit einem alpha-Guajengehalt von 18,4 % erhalten.

### Beispiel 1b: Dehydratisierung von Guajakholzöl mit Weinsäure

Es werden 2250 g Guajakholzöl, 112,5 g Weinsäure und 750 g PEG 400 vorgelegt und auf 210°C aufgeheizt. Ab 180°C entsteht Reaktionswasser, welches kontinuierlich abdestilliert wird. Nach 2 h werden nochmals 112,5 g Weinsäure nachgegeben und für weitere 1,5 h bei 210°C gerührt. Durch Destillation des Reaktionsgemisches bei einer Sumpftemperatur von 125°C und einem Vakuum von 10,0 mbar werden 1426 g einer Fraktion mit einem alpha-Guajengehalt von 24,3 % erhalten.

### Beispiel 2a: Oxidation der Guajen-haltigen Fraktion aus Beispiel 1a

Die Destillationsfraktion aus Beispiel 1a wird für 27 Stunden bei 100°C mit einer Luftmenge von 200 L/h über eine Glasfritte begast. Anschließend wird für 1 h bei 120 °C ohne weitere Luftzufuhr gerührt, um entstandene Peroxide vor der Destillation zu zerstören. Nach diesem Erhitzungsschritt liegt die Peroxidzahl (POZ) bei < 30 meq O/kg und die Mischung wird mit 430 g Triethylcitrat sowie 300 g PEG 600 versetzt und über eine 30 cm Vigreuxkolonne destilliert. Insgesamt werden 850 Gramm einer Fraktion mit 0,8 % Rotundon erhalten.

GC-Analyse (semiquantitativ: Standard Tridecan, Korrekturfaktor Triethylcitrat: 2,555): 50,0 % Triethylcitrat, 0,8 % Rotundon, 5,2 % Bulnesen, 3,6 % Guajen, 1,0 % Guajol, 0,7 % Bulnesol.

### Beispiel 2b: Oxidation der Guajen-haltigen Fraktion aus Beispiel 1b

1400 g der Destillationsfraktion aus Beispiel 1b werden für 24 Stunden bei 100°C mit einer Luftmenge von 120 L/h in einer Blasensäule umgesetzt. Dabei wird zur Reaktionskontrolle der Gehalt an Rotundon und Guajen durch gaschromatographische Analyse bestimmt. Anschließend wird für 2 h bei 120 °C ohne weitere Luftzufuhr gerührt, um entstandene Peroxide vor der Destillation zu zerstören. Nach diesem Erhitzungsschritt liegt die POZ bei < 30 meq O/kg. Anschließend werden 700 g der Reaktionsmischung mit 500 g Triethylcitrat und 500 g PEG 400 versetzt und über eine 30 cm Vigreuxkolonne fraktioniert destilliert. Die Fraktion enthaltend oder bestehend aus der erfindungsgemäßen Rotundon-haltigen Mischung wurde bei einer Temperatur von 130°C - 135°C bei einem Vakuum von 0,2 - 0,8 mbar abgenommen. Es wurden insgesamt 252,2 g der Rotundon-haltigen Mischung erhalten.

GC-Analyse (semiquantitativ: Standard Tridecan, Korrekturfaktor Triethylcitrat: 2,555): 69,2 % Triethylcitrat, 1,0 % Rotundon, 0,2 % Bulnesen, 0,01 % Guajen, 2,0 % Guajol, 1,4% Bulnesol.

### Erfindungsgemäße Halbfertigwaren und Zubereitungen

### Anwendungsbeispiel 1: Verbesserung von Citrus Ölen

| | **Typ Limette** | **Typ Mandarine** | **Typ Orange** | **Typ Grapefruit** | **Typ Zitrone** |
|---|---|---|---|---|---|
| Limettenöl dest. | Q.S. | | | | |
| Mandarinenöl rot italienisch | | Q.S. | | | |
| Orangenöil brasilianisch | | 30,000 | Q.S. | Q.S. | |
| Orangenessenzöl Florida | | | 50,000 | | |
| Grapefruitöl weiß | | | | 60,000 | |
| Zitronenöl italienisch | | | | | Q.S. |
| TERPINEOL, ALPHA- | 25,000 | 0,200 | 0,400 | 0,150 | 1,000 |
| TERPINOLEN | 5,000 | 1,000 | | 0,010 | 1,000 |
| TERPINEOL, GAMMA- | 4,000 | | | | |
| TERPINEN, GAMMA- | 3,000 | 15,000 | | | 16,000 |
| CARYOPHYLLEN | 2,500 | 0,100 | 0,040 | 0,400 | 1,500 |
| NERYLACETAT | 2,500 | | | | 3,000 |
| TERPINENOL, 4- | 4,000 | 0,050 | 0,100 | | 0,100 |
| FENCHOL | 2,000 | | | | |
| GERANYLACETAT | 0,050 | | | 0,080 | 1,600 |
| CYMENE, P- | 1,000 | 1,000 | | 0,200 | 0,200 |
| Citral | 0,500 | | 0,500 | 0,200 | 20,000 |
| LINALOOL | 0,500 | 0,100 | 3,000 | 0,400 | 0,500 |
| VALENCENE Fraktion ex Orange | | | 1,000 | 0,300 | 0,500 |
| EUCALYPTOL | 0,500 | | | | 0,200 |
| ALDEHYD C12 | 0,300 | | 0,200 | 0,050 | 0,000 |
| CARYOPHYLLENOXID | 0,300 | | 0,100 | | |
| CAPRINSÄURE | | | | 0,100 | 0,200 |
| CEDREN nat. | 0,200 | | | | |
| OCIMEN | 0,200 | | | 0,500 | 0,500 |
| Campheröll | 0,100 | | | | 0,050 |
| ALDEHYD C10 | 0,200 | 0,050 | 1,000 | 0,600 | 0,200 |
| ALKOHOL C 10 | 0,100 | 0,010 | 0,080 | 0,020 | 0,010 |
| BORNEOL ISO- | 0,100 | | | | |
| Borneol | 0,050 | | | | 0,020 |
| NEROL | 0,060 | 0,010 | 0,050 | | 0,400 |
| CARVEOL L TRANS | 0,050 | 0,010 | 0,250 | 0,040 | |
| MYRCEN | | 1,800 | 1,800 | 1,800 | 0,300 |
| PINEN, BETA- | | 1,500 | | 0,250 | 0,250 |
| PHELLANDREN ALPHA | 0,050 | 0,050 | 1,000 | 0,050 | |
| HEXENAL, 2E- | | | 0,020 | | |
| ALKOHOL C 6 | | | 0,010 | | |
| ALDEHYD C 7 | | | | | |
| ALDEHYD C 6 | | | 0,100 | | |
| ETHYL METHYLBUTYRAT-2 | | | 0,020 | | |
| ETHYLBUTYRAT | | | 0,200 | | |
| METHYLBUTYRAT | | | | | |
| ETHYLCAPRONAT | | | | | |
| OCTYLACETAT, 1- | | | 0,070 | 0,100 | 0,020 |
| LINALOOLOXID | | | | | |
| ETHYLCAPRYLAT | | | 0,020 | | |
| ALKOHOL C 8 | | 0,050 | 0,500 | 0,200 | |
| HEXENOL, 3Z- | | | | | |
| HEPTYLACETAT | | | | 0,040 | |
| OCTANAL, 6-METHYL- | | 0,000 | | 0,050 | 0,000 |
| ALDEHYD C 9 | | 0,010 | 0,200 | 0,100 | 0,400 |
| CITRONELLAL | | 0,010 | 0,150 | 0,050 | 0,500 |
| ALKOHOL C 7 | | | | | |
| PINEN, ALPHA- | | 3,000 | | 1,000 | 0,500 |
| FENCHON | 0,200 | | | | |
| ALDEHYD C 8 | | 0,100 | 1,000 | 0,500 | 0,020 |
| PHELLANDREN BETA | | 1,000 | | | 0,700 |
| CAREN, DELTA-3- | | | | | |
| CAMPHEN | 0,020 | | | | |
| SINENSAL Fraktion ex Orange | | | 0,500 | | |
| THYMOL | | 0,100 | | | |
| CAPRYLSÄURE | | | 0,100 | 0,050 | 0,050 |
| DODECENAL, 2E- | | 0,010 | | | |
| PERILLA ALDEHYD Ersatz | | 0,050 | 0,150 | 0,040 | 0,100 |
| CITRONELLOL | | 0,010 | 0,070 | | 0,050 |
| ALKOHOL C 9 | | | 0,100 | | 0,050 |
| DIMETHYLANTHRANILAT | | 0,500 | | | |
| CARVYLACETAT, TRANS- | | | | 0,050 | |
| NOOTKATON | | | 0,100 | 0,400 | |
| ALDEHYD C11 | | | | | 0,050 |
| CITRONELLYLACETAT | | | | | 0,130 |
| PALMITINSÄURE | | | | 0,080 | 0,350 |
| DECADIENAL, 2E,4E- | | | | | |
| GERANIOL | | | 0,030 | 0,020 | 0,200 |
| LINALYLACETAT | | | | | |
| DECENAL, 2E- | | | 0,020 | | |
| Menthenthiol-1,8 1% in Limonen | | | | 0,700 | |
| HOTRIENOL | | | | | 0,020 |
| **Mischung aus Bsp. 2b** | 0,007 oder 0,020 oder 0,060 oder 0,140 | 0,010 oder 0,050 oder 0,100 oder 0,150 | 0,004 oder 0,008 oder 0,025 oder 0,050 | 0,04 oder 0,10 oder 0,30 oder 0,50 | 0,004 oder 0,008 oder 0,025 |
| Summe | 100,0 | 100,0 | 100,0 | 100,0 | 100,0 |

Die erhaltenen Öle werden vorzugsweise in einer Dosierung von 10 - 100 ppm, besonders bevorzugt 20 - 50 ppm, in Nahrungsmitteln und Getränken eingesetzt. Für alle Öle wurde durch Zugabe der in Bsp. 2b erhaltenen Mischung der Impact deutlich erhöht und es traten weitere positive Verschiebungen im gesamten Aromenprofil auf. Im Falle des Limettenöls werden die campherartigen Noten verstärkt und das Profil bekommt gewürzartige Noten. Im Falle des Mandarinenöls werden die reifen Noten etwas unterdrückt und die schaligen, frischen Aspekte verstärkt. Für das Orangenöl führt der Zusatz der in Bsp. 2b erhaltenen Mischung zu einem volleren Profil mit mehr reifen und fruchtigen Noten. Für Grapefruitöl treten nach Zugabe der in Bsp. 2b erhaltenen Mischung die holzigen Noten stärker hervor, außerdem wird das Profil fruchtiger und die Schwefelnoten werden verstärkt. Für das Zitronenöl wird das gesamte Profil verstärkt sowie frische und blumige Noten hervorgehoben.

### Anwendungsbeispiel 2: Haselnussaroma

| | |
|---|---|
| ACATHYLPYRIDIN | 5,00 |
| BENZALDEHYD DD | 1,00 |
| CAPRINSÄURE NAT. | 0,50 |
| DECALACTON DELTA | 0,20 |
| DIMETHYLPYRAZIN-2,3 | 0,40 |
| DIMETHYLPYRAZIN-2,5 | 0,40 |
| FILBERTONE | 0,10 |
| FURANEOL 15% ALC | 10,00 |
| ANISALDEHYD | 0,20 |
| METHOXYMETHYLPYRAZIN-2,3 | 0,20 |
| METHYLCYCLOPENTENOLON-3,2,2 | 0,05 |
| PENTANDION-2,3 | 1,00 |
| HELIOTROPIN/PIPERONAL NAT. | 0,50 |
| HEXENOL CIS-3 | 0,50 |
| ISOBUTYRALDEHYD | 2,00 |
| MALTOL | 1,00 |
| METHYLETHYLPYRAZIN-2,3 | 1,00 |
| NONENAL TRANS-2 1% | 0,20 |
| PHENYLETHYLALCOHOL | 0,50 |
| TRIMETHYLPYRAZIN-2,3,5 | 0,50 |
| VANILLE EXTRAKT | 10,00 |
| HAZELNUSS DISTILLAT 3-f. 60 VP | 100,00 |
| **Mischung aus Bsp. 2b** | 0,02 oder 0,06 oder 0,2 oder 0,4 |
| ETHANOL 95% | Q.S. |
| Summe | 1000,00 |

Zur Beurteilung werden typischerweise 80 g Zucker mit 0,1 g des Haselnussaromas versetzt und auf 1 L mit Wasser verdünnt. Im Vergleich zu dem Muster ohne die erfindungsgemäße Mischung aus Bsp. 2b wird der Impact deutlich verstärkt, das Profil abgerundet und die Nuss-Nougat-Note tritt deutlicher hervor.

### Anwendungsbeispiel 3: Erdbeeraroma

| | |
|---|---|
| ESSIGSÄURE | 5 |
| ACETYL METHYL CARBINOL | 1 |
| BUTTERSÄURE | 5 |
| CAPROINSÄURE NAT. FÜR SÜSS & FRUCHTIG | 10 |
| CAPRYLSÄURE | 2 |
| DECALACTON GAMMA | 4 |
| DIMETHYLANTHRANILAT EXTRA | 0,2 |
| ETHYLBUTYRAT | 20 |
| ETHYLCAPROAT | 5 |
| ETHYLMETHYLBUTYRAT-2 | 5 |
| FURANEOL 15% PG | 100 |
| HEDION | 0,2 |
| HEXENOL CIS-3 | 10 |
| MALTOL | 3 |
| METHYLCINNAMAT | 4 |
| METHYLBUTTERSÄURE-2 | 8 |
| METHYLPENTENSÄURE-2,2 | 2 |
| ERDBEER RECOVERY | 50 |
| **Mischung aus Bsp. 2a** | 0,002 oder 0,005 oder 0,01 oder 0,03 |
| ETHYLALKOHOL 96,5% VOL | Q.S. |
| Summe | 1000,0 |

Zur Beurteilung werden typischerweise 80 g Zucker, 1,5 g Zitronensäure mit 0,4 g des Erdbeeraromas versetzt und auf 1 L mit Wasser verdünnt. Im Vergleich zu dem Muster ohne die erfindungsgemäße Mischung aus Bsp. 2a wird der Impact deutlich verstärkt, die Esternoten treten stärker hervor und das Profil bekommt insgesamt eine reifere an Waldfrucht erinnernde Note.

### Anwendungsbeispiel 4: Himbeeraroma

| | |
|---|---|
| ACETALDEHYD 50% | 5 |
| ALKOHOL C 6 | 0,5 |
| ALDEHYD C 6 | 1 |
| BUTTERSÄURE | 0,1 |
| CAPRONSÄURE | 0,1 |
| DAMASCENON | 0,5 |
| DAMASCON BETA | 0,5 |
| ESSIGSÄURE E260 | 20 |
| ETHYLACETAT | 0,3 |
| ETHYLBUTYRAT | 0,3 |
| ETHYLCAPRONAT | 0,3 |
| FRAMBINON^{®} | 5 |
| FURANEOL 15% PG | 25 |
| GERANIOL | 0,25 |
| HEXENAL TRANS-2 | 0,5 |
| HEXENOL CIS-3 | 25 |
| HEXENYLACETAT CIS-3 | 1 |
| HEXYLACETAT | 1 |
| IONON ALPHA | 2 |
| IONON BETA FG | 2 |
| ISOAMYLACETAT | 0,5 |
| ISOBUTYLACETAT | 1 |
| MACROLIDE^{®} SUPRA 1% PG | 0,5 |
| MALTOL | 5 |
| PHENYLETHYLALKOHOL | 0,2 |
| HIMBEER DESTILLAT | 150 |
| **Mischung aus Bsp. 2a** | 0,002 oder 0,005 oder 0,01 oder 0,03 |
| PROPYLENEGYLCOLE | Q.S. |
| Summe | 1000,0 |

Zur Beurteilung werden typischerweise 80 g Zucker, 1,5 g Zitronensäure mit 0,5 g des Himbeeraromas versetzt und auf 1 L mit Wasser verdünnt. Im Vergleich zu dem Muster ohne die erfindungsgemäße Mischung aus Bsp. 2a wird der Impact deutlich verstärkt, die blumigen, an lonon erinnernden Noten treten stärker hervor und das Profil bekommt einen holzigeren Charakter.

### Anwendungsbeispiel 5: Mangoaroma (Typ tropisch)

| | |
|---|---|
| ACETYLMETHYLCARBINOL | 0,2 |
| ALLYLCAPROAT | 3 |
| BUCCOLEAVE ÖL | 0,1 |
| BUTTERSÄURE | 0,1 |
| CAPRYLSÄURE | 1 |
| CARYOPHYLLEN | 2 |
| DECALACTON DELTA | 1 |
| DECALACTON GAMMA | 3 |
| DIPHENYLOXID | 0,5 |
| DMS/DIMETHYLSULFID | 1 |
| ETHANTHIOL 1% TRIA | 0,02 |
| ETHYLACETAT | 2 |
| ETHYLBUTYRAT | 15 |
| ETHYLCAPROAT | 1 |
| ETHYLCAPRYLAT | 0,5 |
| ETHYLMETHYLBUTYRAT-2 | 2 |
| ETHYLVALERAT | 1 |
| FURANEOL 15% PG | 5 |
| HEXENOL CIS-3 | 2 |
| HEXENYLACETAT CIS-3 | 1 |
| HEXYLCAPROAT | 0,5 |
| IONON BETA | 0,02 |
| ISOPROPYLMETHYLTHIAZOL-2,4 1% TRI | 0,1 |
| LINALOOL | 0,5 |
| MALTOL | 5 |
| THIOHEXANOL-3 1% | 0,01 |
| THIOHEXYLACETAT 1% | 0,01 |
| MYRCEN | 1 |
| OCIMEN | 1 |
| OCTALACTON GAMMA | 0,5 |
| ORANGENÖL FLORIDA | 1 |
| TERPINENOL-4 NAT. | 0,1 |
| TERPINOLEN | 0,5 |
| THIOMENTHANON-8,3 1% TRIA | 2 |
| MANGO RECOVERY | 30 |
| **Mischung aus Bsp. 2b** | 0,005 oder 0,02 oder 0,05 oder 0,1 |
| PROPYLENGLYCOL-1,2 | Q.S. |
| Summe | 1000,0 |

Zur Beurteilung werden typischerweise 80 g Zucker, 1,5 g Zitronensäure mit 0,5 g des Mangoaromas versetzt und auf 1 L mit Wasser verdünnt. Im Vergleich zu dem Muster ohne die erfindungsgemäße Mischung aus Bsp. 2b wird der Impact deutlich verstärkt, das gesamte Profil wirkt stärker, charakteristischer und hat mehr schalige, terpenige Noten, besonders treten allerdings die schwefligen Noten stärker hervor.

### Anwendungsbeispiel 6: Birnenaroma

| | |
|---|---|
| ACETALDEHYD | 10 |
| ALKOHOL C6 | 20 |
| ESSIGSÄURE | 2 |
| BUTYLACETAT | 5 |
| ETHYLACETAT | 5 |
| ETHYLBUTYRAT | 3 |
| ETHYLCAPRYLAT | 0,5 |
| ETHYLDECADIENOAT | 1 |
| FURANOL 15% PG | 1 |
| GERANYLBUTYRAT | 1 |
| HEXENAL TRANS-2 | 10 |
| HEXYLACETAT | 20 |
| HEXENYLACETAT CIS-3 | 15 |
| ISOAMYLACETAT | 20 |
| ISOBUTYLACETAT | 5 |
| METHYLMETHYLBUTYRAT | 10 |
| BIRNENSAFT KONZ. 70BX KLAR | 100 |
| BIRNEN WILLIAMS BRANDY 40%VOL | 200 |
| ETHYLALKOHOL 96,5% VOL | 200 |
| **Mischung aus Bsp. 2b** | 0,02 oder 0,04 oder 0,1 oder 0,2 |
| PROPYLENGLYCOL-1,2 | Q.S. |
| Summe | 1000,0 |

Zur Beurteilung werden typischerweise 80 g Zucker, 1,5 g Zitronensäure mit 0,1 g des Bananenaromas versetzt und auf 1 L mit Wasser verdünnt. Im Vergleich zu dem Muster ohne die erfindungsgemäße Mischung aus Bsp. 2b wird der Impact deutlich verstärkt, das die esterigen, reifen Noten treten stärker hervor und das Aroma erinnert mehr an Williams Christ Birnen.

### Anwendungsbeispiel 7: Bananenaroma

| | |
|---|---|
| ACETYLMETHYLCARBINOL | 1 |
| BUTYLACETAT | 1 |
| BUTTERSÄURE | 0,5 |
| ZIMTALDEHYD | 0,3 |
| CITRAL FF | 0,1 |
| ETHYLACETAT | 0,5 |
| ETHYLBUTYRAT | 0,5 |
| EUGENOL NAT. | 0,2 |
| FURANEOL 15% | 3 |
| GERANYLACETAT | 0,04 |
| HEXENAL TRANS-2 | 2 |
| ISOAMYLACETAT MIX | 20 |
| ISOAMYLALKOHOL | 5 |
| ISOAMYLBUTYRAT | 5 |
| ISOAMYLISOVALERAT | 2 |
| ISOBUTYLACETAT | 1 |
| METHYL HEPTIN CARBONAT | 0,05 |
| NONADIENAL TRANS,CIS-2,6 1% | 0,02 |
| ORANGENÖL | 0,2 |
| VANILLIN | 0,5 |
| BANANEN RECOVERY | 50 |
| **Mischung aus Bsp. 2a** | 0,004 oder 0,008 oder 0,02 |
| PROPYLENGLYCOL-1,2 | Q.S. |
| Summe | 1000,0 |

Zur Beurteilung werden typischerweise 80 g Zucker mit 0,4 g des Birnenaromas versetzt und auf 1 L mit Wasser verdünnt. Im Vergleich zu dem Muster ohne die erfindungsgemäße Mischung aus Bsp. 2a wird der Impact deutlich verstärkt und die holzigen, eugenol-artigen Aspekte werden hervorgehoben.

### Anwendungsbeispiel 8: Schokoladenaroma

| | |
|---|---|
| ACETYLMETHYLCARBINOL | 2 |
| ANISALDEHYD 1% | 0,5 |
| BENZALDEHYD 10% | 0,2 |
| BUTTERSÄURE | 0,05 |
| ZIMTALDEHYD | 0,1 |
| CINNAMYLACETAT | 1 |
| CRESOL PARA 0, 1% | 0,2 |
| DECALACTON DELTA | 0,2 |
| DIMETHYLETHYLPYRAZIN-3,5(6),2 | 0,2 |
| DIMETHYLPYRAZIN-2,3 | 0,2 |
| DIMETHYLPYRAZIN-2,5 | 0,1 |
| DIMETHYLPYRAZIN-2,6 | 0,05 |
| DODECALACTON DELTA | 0,1 |
| ETHYLCAPROAT | 0,05 |
| FURANEOL 15% | 5 |
| GUAJACOL 1% | 0,5 |
| ISOAMYLALKOHOL | 2 |
| ISOBUTYRALDEHYD | 0,5 |
| ISOVALERIANSÄURE NAT. | 0,1 |
| ISOVALERIANALDEHYD | 2 |
| METHYLCYCLOPENTENOLON-3,2,2 | 2 |
| METHYLGUAJACOL-4 | 0,1 |
| METHYLPHENYLHEXENAL TRANS-5,2,2 | 2 |
| METHYLPYRAZIN-2 | 0,1 |
| PHENYLACETALDEHYD | 1 |
| PHENYLESSIGSÄURE | 2 |
| ETHYLPHENYLACETAT | 0,5 |
| PHENYLETHYLALKOHOL | 0,5 |
| TRIMETHYLPYRAZIN-2,3,5 | 0,5 |
| VANILLE EXTRAKT BOURBON 3X | 5 |
| VANILLIN | 50 |
| CACAO EXTRAKT | 50 |
| TRIACETIN | 250 |
| **Mischung aus Bsp. 2b** | 0,004 oder 0,02 oder 0,04 |
| PROPYLENGLYCOL-1,2 | 621,25 |
| Summe | 1000,0 |

Zur Beurteilung werden typischerweise 80 g Zucker mit 0,5 g des Schokoladenaromas versetzt und auf 1 L mit Wasser verdünnt. Im Vergleich zu dem Muster ohne die erfindungsgemäße Mischung aus Bsp. 2b wird der Impact deutlich verstärkt, außerdem wirkt das Aroma voller und komplexer und die phenolischen Noten werden stärker betont.

### Anwendungsbeispiel 9: Ananasaroma

| | |
|---|---|
| ACETALDEHYD NAT. 50%TRIC | 5 |
| ESSIGSÄURE | 5 |
| ALLYLCAPROAT | 5 |
| CAPRONSÄURE | 0,5 |
| ETHYLBUTYRAT | 5 |
| ETHYLCAPROAT | 8 |
| ETHYLISOVALERAT | 2 |
| ETHYLMETHYLTHIOPROPIONAT-3 | 0,5 |
| FURANEOL 15% PG | 20 |
| HEXENOL CIS-3 | 0,5 |
| ISOAMYLACETAT | 5 |
| ISOAMYLISOVALERAT | 2 |
| METHYLMETHYLTHIOPROPIONAT-3 NAT. | 1 |
| ANANAS RECOVERY KONZ. | 50 |
| **Mischung aus Bsp. 2b** | 0,007 oder 0,03 oder 0,07 |
| PROPYLENGLYCOL | Q.S. |
| Summe | 1000,0 |

Zur Beurteilung werden typischerweise 80 g Zucker, 1,5 g Zitronensäure mit 0,3 g des Ananasaromas versetzt und auf 1 L mit Wasser verdünnt. Im Vergleich zu dem Muster ohne die erfindungsgemäße Mischung aus Bsp. 2b wird der Impact deutlich verstärkt, außerdem wirkt das Aroma voller und komplexer und die esterigen Noten werden stärker betont.

### Anwendungsbeispiel 10: Rumaroma

| | |
|---|---|
| ACETALDEHYD DIETHYLACETAL | 25 |
| ACETALDEHYD NATÜRLICH | 25 |
| ESSIGSÄURE | 50 |
| ACETOIN | 1 |
| BUTTERSÄURE | 1 |
| CAPRINSÄURE | 1 |
| CAPRONSÄURE | 0,1 |
| DAMASCENON BETA | 0,05 |
| ETHYLACETAT | 50 |
| ETHYLALKOHOL 96,5% VOL | 80 |
| ETHYLCAPRYLAT | 0,6 |
| ETHYLFORMAT | 10 |
| ETHYLBUTYRAT | 0,1 |
| EUGENOL | 0,05 |
| GUAJACOL | 0,02 |
| ISOBUTANOL | 50 |
| ISOVALERALDEHYD | 0,5 |
| ISOVALERINSÄURE | 0,5 |
| METHYLBUTANOL-3 | 150 |
| PHENYLETHYLALKOHOL | 2 |
| EICHENHOLZ EXTRAKT | 10 |
| RUM JAMAICA 75% VOL | 100 |
| VANILLE EXTRAKT | 5 |
| **Mischung aus Bsp. 2b** | 0,005 oder 0,01 oder 0,075 oder 0,2 |
| WASSER | Q.S. |
| Summe | 1000,0 |

Zur Beurteilung werden typischerweise 20 g Zucker, 2 g Glycerin, 1 g Karamellsirup sowie 417 ml Ethanol (96,5%) mit 1,2 g des Rumaromas versetzt und auf 1 L mit Wasser verdünnt. Im Vergleich zu dem Muster ohne die erfindungsgemäße Mischung aus 2b wird der Impact deutlich verstärkt, außerdem wirkt das Aroma voller und komplexer, die Eichenholznoten werden Verstärkt und das gesamte Profil erinnert mehr an fassgelagerte Qualitäten.

### Anwendungsbeispiel 11: Whiskeyaroma

| | |
|---|---|
| Caprinsäure | 130 |
| Caprylsäure | 10 |
| Ethylacetat | 100 |
| Ethylcaprinat | 50 |
| Ethylcapronat | 2 |
| Ethylcaprylat | 10 |
| Isobutanol | 248 |
| Methylbutanol-3 | 198 |
| Ethylalkohol | 247,98 |
| Weinhefenöl | 4 |
| **Mischung aus Bsp. 2b** | 0,02 |
| Summe | 1000,0 |

Durch Aufsatz von 0,005 oder 0,01 % auf einen vorhandenen Whiskey wird der Impact deutlich verstärkt, außerdem wirkt das Aroma voller und runder, die Eichenholznoten werden verstärkt und das gesamte Profil erinnert mehr an fassgelagerte Qualitäten.

### Anwendungsbeispiel 12: Muskatnussaroma

| | |
|---|---|
| Eugenol | 5 |
| Linalool | 12 |
| Pinen alpha | 180 |
| Pinen beta | 50 |
| Terpineol alpha | 2,5 |
| Muskatnussöl | 50 |
| **Ethylalkohol** | 700,45 |
| **Mischung aus Bsp. 2a** | 0,05 |
| Summe | 1000,0 |

### Anwendungsbeispiel 13: Pfefferaroma

| | |
|---|---|
| Pfefferöl schwarz | 10 |
| Piperin | 0,5 |
| Ethylalkohol | 989,4 |
| **Mischung aus Bsp. 2b** | 0,1 |

Dieses Pfefferaroma kann in Dosierungen von 0,05 oder 0,1% eingesetzt werden. Im Vergleich zu dem Muster ohne die erfindungsgemäße Mischung wird der Impact deutlich verstärkt, außerdem wirkt das Aroma typischer und frischer.

### Anwendungsbeispiel 14: Zwiebelaroma

| | |
|---|---|
| DIMETHYLSULFID | 2,00 |
| DIPROPYLDISULFID | 13,00 |
| ISOBUTYRALDEHYD | 4,00 |
| METHYLPROPYLDISULFID | 7,00 |
| ZWIEBELÖL | 2,00 |
| THIOPENTANON-3,2 | 3,00 |
| **Mischung aus Bsp. 2b** | 0,01 oder 0,05 oder 0,10 |
| TRIACETIN | Q.S. |
| Summe | 1000,00 |

Zur Beurteilung werden typischerweise 3 g Salz mit 0,1 g des Zwiebelaromas versetzt und auf 1 L mit warmem Wasser aufgefüllt. Im Vergleich zu dem Muster ohne die erfindungsgemäße Mischung aus Bsp. 2b wird der Impact deutlich verstärkt, außerdem wirkt das Aroma frischer und ausbalancierter.

### Anwendungsbeispiel 15: Kartoffelaroma

| | |
|---|---|
| ACETYLMETHYLCARBINOL | 20,00 |
| ACETYLTHIAZOL-2 | 2,00 |
| ALDEHYD C 6 | 2,00 |
| ALDEHYD C14 | 6,00 |
| ALKOHOL C 6 | 4,00 |
| BUTTERSäURE | 5,00 |
| DECADIENAL TRANS,TRANS-2,4 | 0,10 |
| DIMETHYLPYRAZIN-2,3 | 2,00 |
| DIMETHYLSULFID | 5,00 |
| DODECALACTON DELTA | 8,00 |
| HEPTANON-2 | 2,00 |
| HEPTENAL TRANS-2 | 0,50 |
| ISOBUTYRALDEHYD | 10,00 |
| ISOPROPYLMETHOXYPYRAZIN-2,3 0.1% TRIA | 10,00 |
| METHOXYETHYLPYRAZIN-2,3 0.1% TRIA | 10,00 |
| METHYLGUAJACOL-4 | 0,20 |
| METHYLMERCAPTAN | 0,10 |
| METHYLTHIOPROPANAL-3 | 4,00 |
| OCTENOL-1,3 | 0,10 |
| PHENYLACETALDEHYD | 0,50 |
| **Mischung aus Bsp. 2b** | 0,01 oder 0,05 oder 0,10 |
| TRIACETIN | Q.S. |
| Summe | 1000,0 |

Zur Beurteilung werden typischerweise 3 g Salz mit 0,2 g des Kartoffelaromas versetzt und auf 1 L mit warmem Wasser aufgefüllt. Im Vergleich zu dem Muster ohne die erfindungsgemäße Mischung wird der Impact deutlich verstärkt, außerdem wirkt das gesamte Aroma intensiver.

### Anwendungsbeispiel 16: Pilzaroma

| | |
|---|---|
| ALKOHOL C 8 | 2,00 |
| BENZALDEHYD DD | 1,00 |
| CAPRINSÄURE | 5,00 |
| CAPRYLSÄURE | 4,00 |
| FENCHOL | 0,10 |
| FURFURYLALKOHOL | 1,50 |
| ISOVALERIANSAEURE | 0,50 |
| METHYLTHIOPROPANAL-3 | 0,10 |
| OCTANOL-3 | 0,60 |
| OCTENOL-1,3 | 40,00 |
| OCTENON-1,3 | 0,40 |
| OCTENYLACETAT-1,3 | 0,60 |
| PILZ (STEIN-)-EXTRAKT | 5,00 |
| TERPINENOL-4 | 8,00 |
| TRIMETHYLPYRAZIN-2,3,5 | 1,00 |
| **Mischung aus Bsp. 2b** | 0,01 oder 0,05 oder 0,10 |
| TRIACETIN | Q.S. |
| Summe | 1000,0 |

Zur Beurteilung werden typischerweise 3 g Salz mit 0,2 g des Pilzaromas versetzt und auf 1 L mit warmem Wasser aufgefüllt. Im Vergleich zu dem Muster ohne die erfindungsgemäße Mischung aus Bsp. 2b wird der Impact deutlich verstärkt, außerdem werden holzige und erdige Noten verstärkt und das gesamte Aroma verschiebt sich in Richtung Wildpilz.

### Anwendungsbeispiel 17: Karottenaroma

| | |
|---|---|
| ACETYLMETHYLCARBINOL | 1,00 |
| ALDEHYD C 6 | 0,10 |
| ALDEHYD C 8 | 0,10 |
| ALDEHYD C10 | 0,10 |
| LORBEERÖL | 3,00 |
| BENZALDEHYD | 1,00 |
| CAPRONSÄURE | 1,00 |
| CAPRYLSÄURE | 2,00 |
| CAREN DELTA-3 | 0,20 |
| CARYOPHYLLEN | 12,00 |
| IONON BETA | 1,20 |
| ISOVALERIANSÄURE | 0,70 |
| KAROTTENSAMENÖL | 5,00 |
| PALMAROSAÖL | 1,00 |
| PHELLANDREN FRAKTION EX EUCALYPTUSÖL | 7,00 |
| PINEN BETA | 1,60 |
| **Mischung aus Bsp. 2a** | 0,02 oder 0,05 oder 0,10 |
| TRIACETIN | Q.S. |
| Summe | 1000,0 |

Zur Beurteilung werden typischerweise 3 g Salz mit 0,4 g des Karottenaromas versetzt und auf 1 L mit warmem Wasser aufgefüllt. Im Vergleich zu dem Muster ohne die erfindungsgemäße Mischung aus Bsp. 2a wird der Impact deutlich verstärkt, außerdem wirkt das Aroma süßer und reifer.

### Anwendungsbeispiel 18: Paprikaaroma (rot)

| | |
|---|---|
| PAPRIKASAFT KONZ. ROT 65BX SAUTIERT | 50,00 |
| DIMETHYLSULFID | 2,00 |
| ETHYLISOBUTYRAT | 3,00 |
| ETHYLMETHYLBUTYRAT-2 | 1,00 |
| FURANEOL | 35,00 |
| GUAJACOL | 0,30 |
| HEXENAL TRANS-2 1% TRIA | 10,00 |
| IONON BETA FG | 2,50 |
| ISOBUTYLMETHOXYPYRAZIN-3,2 1% TRIA | 37,00 |
| ISOPROPYLMETHOXYPYRAZIN-2,3 1% TRIA | 1,00 |
| METHOXYMETHYLPROPYLPYRAZIN 1% TRIA | 12,00 |
| METHYLISOVALERIANAT | 1,50 |
| METHYLMETHYLBUTYRAT-2 | 2,00 |
| PHENYLESSIGSÄURE | 13,00 |
| PROPIONSÄURE | 60,00 |
| **Mischung aus Bsp. 2b** | 0,02 oder 0,05 oder 0,10 |
| TRIACETIN | Q.S. |
| Summe | 1000,0 |

Zur Beurteilung werden typischerweise 3 g Salz mit 0,2 g des Paprikaaromas versetzt und auf 1 L mit warmem Wasser aufgefüllt. Im Vergleich zu dem Muster ohne die erfindungsgemäße Mischung aus Bsp. 2b wird der Impact deutlich verstärkt, außerdem wirkt das Aroma reifer und saftiger und besitzt insgesamt mehr Geschmackstiefe und Komplexität.

### Anwendungsbeispiel 19: Tomatenaroma

| | |
|---|---|
| ALDEHYD C 6 | 2,00 |
| ALKOHOL C 6 | 1,20 |
| CLOVE LEAF OIL | 5,00 |
| DAMASCENON | 0,40 |
| DIMETHYLSULFID | 4,50 |
| ETHYLSALICYLAT | 0,20 |
| FURANEOL | 1,00 |
| GERANYLBUTYRAT | 2,00 |
| GUAJACOL | 0,20 |
| HEXENOL CIS-3 | 0,40 |
| IONON BETA | 1,00 |
| ISOBUTYLTHIAZOL-2 | 0,20 |
| LINALOOL | 0,10 |
| METHYLHEPTENON-6,5,2 | 0,60 |
| PHENYLETHYLALKOHOL | 30,00 |
| Mischung aus Bsp. 2b | 0,01 oder 0,05 oder 0,10 |
| TRIACETIN | Q.S. |
| Summe | 1000,0 |

Zur Beurteilung werden typischerweise 3 g Salz mit 0,1 g des Tomatenaromas versetzt und auf 1 L mit warmem Wasser aufgefüllt. Im Vergleich zu dem Muster ohne die erfindungsgemäße Mischung aus Bsp. 2b wird der Impact deutlich verstärkt, außerdem wirkt das Aroma reifer und saftiger und besitzt insgesamt mehr Geschmackstiefe und wirkt authentischer.

### Anwendungsbeispiel 19a: SprühgetrocknetesTomatenaroma

| | |
|---|---|
| Wasser | 1135,00 |
| Maltodextrin | 665,00 |
| Tomatenaroma aus Anwendungsbeispiel 19 | 200,00 |
| Stärke | 165,00 |

Die Bestandteile werden vermischt und anschließend sprühgetrocknet.

### Anwendungsbeispiel 20: Krauseminze Aroma

| | |
|---|---|
| Menthol | 300 |
| Carvon | 200 |
| Krauseminzöl Typ Nativ | 200 |
| Anethol | 50 |
| Pfefferminzöl Mentha arvensis rektifiziert | 100 |
| Pfefferminzöl Mentha piperita Typ Willamette | Q.S. |
| **Mischung aus Bsp. 2b** | 0,6 |
| **Summe** | 1000,0 |

### Anwendungsbeispiel 21: Kaugummi mit Krauseminze Aroma

| | | |
|---|---|---|
| Kaugummibase | 21 | 30 |
| Glycerin | 0,5 | 1 |
| Glucosesirup | 16,5 | - |
| Puderzucker | Q.S. | - |
| Sorbitol (in Pulverform) | - | Q.S. |
| Palatinit | - | 9,5 |
| Xylitol | - | 2 |
| Mannitol | - | 3 |
| Aspartam | - | 0,1 |
| Acesulfam K | - | 0,1 |
| EmulgumTM (Emulgator) | - | 0,3 |
| Sorbitol 70%, in Wasser | - | 14 |
| Aroma Typ Krauseminze (Anwendungsbeispiel 20) | 1 | 1,4 |
| Summe | 100,0 | 100,0 |

### Anwendungsbeispiel 22: Mundwasser (ohne Alkohol) mit Krauseminze Aroma

| | |
|---|---|
| Cremophor RH 455 | 1,8 |
| Entionisiertes Wasser | Q.S. |
| Sorbitol 70% | 10 |
| Natriumfluorid | 0,18 |
| Natriumsaccharin 450 | 0,1 |
| Solbrol M Natriumsalz | 0,15 |
| Pellitorin-Lösung PLM (enthaltend 10% Pellitorin) | - |
| Aroma Typ Krauseminze (Anwendungsbeispiel 20) | 0,2 |
| Summe | 100,0 |

### Anwendungsbeispiel 23: Mundwasser (mit Alkohol) mit Krauseminze Aroma

| | | |
|---|---|---|
| Ethylalkohol 96% | 10 | 7 |
| Cremophor CO 40 | 1 | 1 |
| Benzoesäure | 0,1 | 0,1 |
| Entionisiertes Wasser | Q.S. | Q.S. |
| Sorbitol 70% | 5 | 5 |
| Natriumsaccharin 450 | 0,07 | 0,05 |
| L-Blue 5000 (1% in Wasser) | 0,1 | 0,1 |
| 1,2-Propylenglycol | - | 3 |
| Cetylpyridiniumchlorid | - | 0,07 |
| Wasserstoffperoxid (35% H₂O₂ in Wasser) | - | 4 |
| Aroma Typ Krauseminze (Anwendungsbeispiel 20) | 0,25 | 0,25 |
| Summe | 100,0 | 100,0 |

### Anwendungsbeispiel 24: Zahnpasta mit Krauseminze Aroma

| Entionisiertes Wasser | 36,39 |
|---|---|
| Glycerin | 20 |
| Solbrol M (Natriumsalz) | 0,15 |
| Natriummonofluorphosphat | 0,76 |
| Saccharin | 0,2 |
| Dicalciumphosphat-Dihydrat | 36 |
| Aerosil^{®} 200 (Silica) | 3 |
| Natriumcarboxymethylcellulose | 1,2 |
| Natriumlaurylsulfat (Texapon) | 1,3 |
| Aroma Typ Krauseminze (Anwendungsbeispiel 20) | 1 |

### Anwendungsbeispiel 25: Bonbon

| | | |
|---|---|---|
| Wasser | 2,75 | 2,5 |
| Zucker | Q.S. | Q.S. |
| Glucosesirup | 36,9 | 36 |
| Maltose | - | 2 |
| Palmkernöl | - | 0,8 |
| Citronensäure | - | 0,25 |
| Ginseng Extrakt | - | 0,4 |
| Blauer Farbstoff | - | 0,01 |
| Aroma Typ Krauseminze (Anwendungsbeispiel 20) | 0,25 | 0,35 |
| Summe | 100,0 | 100,0 |

### Anwendungsbeispiel 26: Tomatencremesuppe

| Tomatenpulver | Q.S. |
|---|---|
| Zucker | 17 |
| Stärke | 16,75 |
| Fettpulver | 13 |
| Salz | 7 |
| Kartoffelgranulat | 5 |
| Michprotein | 2 |
| Hefeextrakt | 1 |
| Olivenöl | 1 |
| Zwiebelpulver | 1 |
| Knoblauchpulver | 0,5 |
| Getrocknete Petersilie | 0,3 |
| Äpfelsäure | 0,06 |
| Ingwerextrakt | 0,15 |
| Basilikumaroma (sprühgetrocknet) | 0,08 |
| Sprühgetrocknete Zusammensetzung Bsp 19a | 0,3 |
| Summe | 100 |

Alle Zutaten werden vermischt. Anschließend werden 120 g dieser Mischung zusammen mit 1000 ml kochendem Wasser aufgegossen.

### Anwendungsbeispiel 27: Instant Tomatensuppe

| Tomatenpulver | Q.S. |
|---|---|
| Zucker | 25 |
| Kartoffelstärke | 23,75 |
| Fettpulver | 7,7 |
| Salz | 4,7 |
| Milchpulver (26% Fett) | 3 |
| Rote Beete Saftkonzentrat (sprühgetrocknet) | 1,22 |
| Zitronensäure (wasserfrei) | 0,73 |
| Hefeextrakt | 0,73 |
| Carotin (10%) Pulver | 0,04 |
| Vitamin B2/Riboflavin Pulver | 0,01 |
| Basilikumaroma (sprühgetrocknet) | 0,01 |
| Sprühgetrocknete Zusammensetzung (Bsp 19a) | 0,3 |
| Summe | 100 |

Alle Zutaten werden vermischt und in Portionsbeutel zu je 24,5 g verpackt. Zur Zubereitung wird der Inhalt eines Beutels in eine Tasse entleert und mit 230 ml kochendem Wasser aufgelöst. Gut umrühren und vor Verzehr einige Minuten warten.

## Patentansprüche

1. Verfahren zur Herstellung einer Mischung umfassend Rotundon, umfassend oder bestehend aus folgenden Schritten:
a) Umsetzen von Holzöl(en), Extrakt(en) und/oder Naturharz(en), bevorzugt von Guajakholzöl, enthaltend Guajol und Bulnesol, bevorzugt enthaltend 15 bis 45 Gew.-% Guajol und 25 bis 55 Gew.-% Bulnesol, besonders bevorzugt enthaltend 20 bis 35 Gew.-% Guajol und 30 bis 45 Gew.-% Bulnesol, mit einer oder mehreren organischen Säure(n),
b) Abtrennen der Guajen-haltigen Fraktion von der in Schritt a) erhaltenen Mischung, bevorzugt durch Destillation,
c) Oxidation der abgetrennten Guajen-haltigen Fraktion aus Schritt b), bevorzugt mit Umgebungsluft und/oder reinem Sauerstoff als Oxidationsmittel,
d) optional: Erhitzen der in Schritt c) erhaltenen oxidierten Mischung, bevorzugt Erhitzen über einen Zeitraum von 1 bis 3 h und/oder bei 110 bis 150 °C, vorzugsweise bei 115 bis 125 °C,
e) Abtrennen der Rotundon-haltigen Fraktion von der in Schritt c) oder d), falls vorhanden, erhaltenen Mischung, bevorzugt durch Destillation, um die Rotundon-haltige Mischung zu erhalten.

2. Verfahren nach Anspruch 1, wobei die in Schritt a) eingesetzte(n) organische(n) Säure(n) ausgewählt ist bzw. sind aus der Gruppe bestehend aus Citronensäure, Äpfelsäure, Weinsäure und Oxalsäure, vorzugsweise wobei die in Schritt a) eingesetzte organische Säure Weinsäure ist.

3. Verfahren nach einem der vorangehenden Ansprüche, wobei in Schritt a) das Gewichtsverhältnis von Holzöl(en), Extrakt(en) und/oder Naturharz(en) enthaltend Guajol und Bulnesol zu organischer Säure bzw. zu organischen Säuren zwischen 50:1 und 3:1, bevorzugt zwischen 20:1 und 2:1, besonders bevorzugt zwischen 12:1 und 8:1 liegt.

4. Verfahren nach einem der vorangehenden Ansprüche, wobei die Umsetzung in Schritt a) in Gegenwart eines Co-Solvenzes oder mehrerer Co-Solvenzien, bevorzugt in Gegenwart eines Polyethylenglycols oder mehrerer Polyethylenglycole, besonders bevorzugt in Gegenwart von PEG 600, stattfindet und vorzugsweise wobei das Gewichtsverhältnis von Holzöl(en), Extrakt(en) und/oder Naturharz(en) enthaltend Guajol und Bulnesol zu dem einen oder den mehreren Co-Solvenz(ien) 4:1 bis 1:2 beträgt.

5. Verfahren nach einem der vorangehenden Ansprüche, wobei die Reaktionstemperatur der Umsetzung in Schritt a) zwischen 160 und 260 °C, bevorzugt zwischen 180 und 220 °C, liegt und/oder die Reaktionszeit der Umsetzung in Schritt a) zwischen 1 und 20 h, bevorzugt zwischen 5 und 15 h, liegt und/oder die Umsetzung in Schritt a) bei Normaldruck oder bei Unterdruck erfolgt, vorzugsweise bei 400 bis 900 mbar, besonders bevorzugt bei 500 bis 700 mbar.

6. Verfahren nach einem der vorangehenden Ansprüche, wobei in Schritt b) das Abtrennen der Guajen-haltigen Fraktion von der in Schritt a) erhaltenen oder bereitgestellten Mischung durch Destillation erfolgt, bevorzugt bei Unterdruck, vorzugsweise bei 1,0 bis 20,0 mbar und einer Sumpftemperatur von 90 bis 175 °C, besonders bevorzugt bei 2,0 bis 10,0 mbar und einer Sumpftemperatur von 105 bis 160 °C, weiter bevorzugt bei 4,0 mbar und einer Sumpftemperatur von 130 °C.

7. Verfahren nach einem der vorangehenden Ansprüche, wobei in Schritt c) die Oxidation der Guajen-haltigen Fraktion aus Schritt b) mit Umgebungsluft und/oder reinem Sauerstoff als Oxidationsmittel und/oder bei einer Temperatur von 60 bis 150 °C, bevorzugt 80 bis 130 °C, besonders bevorzugt 90 bis 110°C, und/oder mit einer Gasmenge des Oxidationsmittels von ≥ 10 bis ≤ 1000 l/h, bevorzugt von ≥ 30 bis ≤ 500 l/h, besonders bevorzugt von ≥ 40 bis ≤ 100 l/h, pro kg Edukt und/oder über einen Zeitraum von 10 bis 60 h, bevorzugt von 15 bis 30 h, stattfindet.

8. Verfahren nach einem der vorangehenden Ansprüche, wobei die Peroxidzahl der in Schritt c) oder d), falls vorhanden, erhaltenen Mischung < 30 meq O/kg, bevorzugt < 20 meq O/kg beträgt.

9. Verfahren nach einem der vorangehenden Ansprüche, wobei der Mischung in Schritt e) vor und/oder während der Destillation ein oder mehr Schleppmittel ausgewählt aus der Gruppe bestehend aus Triacetin und Triethylcitrat im Gewichtsverhältnis von in Schritt c) oder d), falls vorhanden, erhaltener Mischung zu Schleppmittel von 4:1 bis 1:4, bevorzugt von 3:1 bis 1:1,
und/oder
ein oder mehr Co-Solvenz(ien) ausgewählt aus der Gruppe bestehend aus Polyethylenglycolen und Palatinol Z, bevorzugt PEG 400 und/oder PEG 600, im Gewichtsverhältnis von in Schritt c) oder d), falls vorhanden, erhaltener Mischung zu Co-Solvenz(ien) von 10:1 bis 1:5, bevorzugt von 5:1 bis 1:1,
zugegeben werden.

10. Verfahren nach einem der vorangehenden Ansprüche, wobei in Schritt e) das Abtrennen der Rotundon-haltigen Fraktion von der in Schritt c) oder d), falls vorhanden, erhaltenen Mischung durch Destillation, bevorzugt bei Unterdruck, vorzugsweise bei 3 bis 5 mbar, stattfindet.

11. Rotundon-haltige Mischung, umfassend oder bestehend aus Rotundon, Bulnesen, Guajen, Guajol und Bulnesol sowie optional Triethylcitrat und/oder Triacetin.

12. Verwendung einer Mischung nach Anspruch 11 zum Erzeugen, Vermitteln oder Modifizieren, vorzugsweise Verstärken, eines oder mehrerer Geschmacks- und/oder Geruchseindrücke, vorzugsweise ausgewählt aus der Gruppe bestehend aus Citrus-Frucht-, Mint-, Muskatnuss-, Pfeffer-, Gewürz-, Kräuter-, Kaffee-, Schokolade, Nuss- , Nougat-Nuss-Eindrücken, gelagerte Spirituosen (z.B. Whiskey-, Rum-, Brandy-Eindrücken), Wein- und Savory-Eindrücken.

13. Der Ernährung, der Mundpflege oder dem Genuss dienende oder kosmetische oder zur oralen Aufnahme bestimmte pharmazeutische Zubereitung oder Halbfertigware zur Herstellung einer der Ernährung, der Mundpflege oder dem Genuss dienenden oder kosmetischen oder zur oralen Aufnahme bestimmten pharmazeutischen Zubereitung, umfassend eine Mischung nach Anspruch 11.

14. Zubereitung oder Halbfertigware nach Anspruch 13, zudem umfassend einen oder mehrere (weitere) Geschmacks- und/oder Geruchsstoffe, wobei die Gesamtmenge an Mischung nach Anspruch 11 ausreicht, um einen oder mehrere Geschmacks- und/oder Geruchseindrücke des/der (weiteren) Geschmacks- und/oder Geruchsstoffe zu modifizieren, vorzugsweise zu verstärken.

15. Verfahren zur Herstellung einer Zubereitung oder Halbfertigware nach Anspruch 13 oder 14, umfassend oder bestehend aus folgenden Schritten:
- Bereitstellen einer Mischung nach Anspruch 11 sowie eines oder mehrerer weiterer Bestandteile, wobei der weitere Bestandteil bzw. ein weiterer Bestandteil oder mehrere weitere Bestandteile vorzugsweise ausgewählt ist / sind aus der Gruppe bestehend aus Geschmacks- und/oder Geruchsstoffen, und
- Mischen der Mischung nach Anspruch 11 mit dem bzw. den weiteren Bestandteilen.

## Claims

1. Process for the preparation of a mixture comprising rotundone, comprising or consisting of the following steps:
(a) reacting wood oil(s), extract(s) and/or natural resin(s), preferably guaiac wood oil, containing guaiol and bulnesol, preferably containing 15 to 45% by weight of guaiol and 25 to 55% by weight of bulnesol, more preferably containing 20 to 35% by weight of guaiol and 30 to 45% by weight of bulnesol, with one or more organic acid(s),
b) separating the guaiene-containing fraction from the mixture obtained in step a), preferably by distillation,
c) oxidizing the separated guaiene-containing fraction from step b), preferably with ambient air and/or pure oxygen as oxidizing agent,
d) optionally: heating the oxidized mixture obtained in step c), preferably heating over a period of 1 to 3 h and/or at 110 to 150 °C, preferably at 115 to 125 °C,
e) separating the rotundone-containing fraction from the mixture obtained in step c) or d), if present, preferably by distillation, to obtain the rotundone-containing mixture.

2. Process according to claim 1, wherein the organic acid(s) used in step a) is/are selected from the group consisting of citric acid, malic acid, tartaric acid and oxalic acid, preferably wherein the organic acid used in step a) is tartaric acid.

3. Process according to any of the preceding claims, wherein in step a) the weight ratio of wood oil(s), extract(s) and/or natural resin(s) containing guaiol and bulnesol to organic acid(s) is between 50:1 and 3:1, preferably between 20:1 and 2:1, particularly preferably between 12:1 and 8:1.

4. Process according to any of the preceding claims, wherein the reaction in step a) takes place in the presence of a co-solvent or several co-solvents, preferably in the presence of a polyethylene glycol or several polyethylene glycols, particularly preferably in the presence of PEG 600, and preferably wherein the weight ratio of wood oil(s), extract(s) and/or natural resin(s) containing guaiol and bulnesol to the one or more co-solvent(s) is from 4:1 to 1:2.

5. Process according to any of the preceding claims, wherein the reaction temperature of the reaction in step a) is between 160 and 260°C, preferably between 180 and 220°C, and/or the reaction time of the reaction in step a) is between 1 and 20 h, preferably between 5 and 15 h, and/or the reaction in step a) takes place at normal pressure or at negative pressure, preferably at 400 to 900 mbar, particularly preferably at 500 to 700 mbar.

6. Process according to any of the preceding claims, wherein in step b) the separation of the guaiene-containing fraction from the mixture obtained or provided in step a) is carried out by distillation, preferably at negative pressure, preferably at 1.0 to 20.0 mbar and a sump temperature of 90 to 175°C, particularly preferably at 2.0 to 10.0 mbar and a sump temperature of 105 to 160°C, further preferably at 4.0 mbar and a sump temperature of 130°C.

7. Process according to any of the preceding claims, wherein in step c) the oxidation of the guaiene-containing fraction from step b) is carried out with ambient air and/or pure oxygen as oxidizing agent and/or at a temperature of 60 to 150°C, preferably 80 to 130°C, particularly preferably 90 to 110°C, and/or with a gas quantity of the oxidizing agent of ≥ 10 to ≤ 1000 l/h, preferably of ≥ 30 to ≤ 500 l/h, particularly preferably of ≥ 40 to ≤ 100 l/h, per kg of starting material and/or over a period of 10 to 60 h, preferably of 15 to 30 h.

8. Process according to any of the preceding claims, wherein the peroxide value of the mixture obtained in step c) or d), if present, is < 30 meq O/kg, preferably < 20 meq O/kg.

9. Process according to any of the preceding claims, wherein before and/or during distillation one or more entraining agents selected from the group consisting of triacetin and triethyl citrate in a weight ratio of mixture obtained in step c) or d), if present, to entraining agent of from 4:1 to 1:4, preferably from 3:1 to 1:1,
and/or
one or more co-solvent(s) selected from the group consisting of polyethylene glycols and Palatinol Z, preferably PEG 400 and/or PEG 600, in a weight ratio of the mixture obtained in step c) or d), if present, to co-solvent(s) of 10:1 to 1:5, preferably 5:1 to 1:1,
are added the mixture in step e).

10. Process according to any of the preceding claims, wherein in step e) the separation of the rotundone-containing fraction from the mixture obtained in step c) or d), if present, takes place by distillation, preferably at negative pressure, preferably at 3 to 5 mbar.

11. Rotundone-containing mixture comprising or consisting of rotundone, bulnesene, guaiene, guaiol and bulnesol and optionally triethyl citrate and/or triacetin.

12. Use of a mixture according to claim 11 for producing, imparting or modifying, preferably enhancing, one or more taste and/or odor impressions, preferably selected from the group consisting of citrus, fruit, mint, nutmeg, pepper, spice, herb, coffee, chocolate, nut, nougat-nut impressions, aged spirits (e.g. whiskey, rum, brandy impressions), wine and savory impressions.

13. A nutritional, oral care or edible or cosmetic or oral pharmaceutical preparation or semi-finished product for the manufacture of a nutritional, oral care or edible or cosmetic or oral pharmaceutical preparation, comprising a mixture according to claim 11.

14. Preparation or semi-finished product according to claim 13, further comprising one or more (further) flavouring and/or odour substances, wherein the total amount of mixture according to claim 11 is sufficient to modify, preferably enhance, one or more taste and/or odour impressions of the (further) flavouring and/or odour substances.

15. Process for preparing a preparation or semi-finished product according to claim 13 or 14, comprising or consisting of the following steps:
- Providing a mixture according to claim 11 as well as one or more further ingredients, wherein the further ingredient or one further ingredient or several further ingredients is/are preferably selected from the group consisting of flavouring and/or odorous substances, and
- mixing the mixture according to claim 11 with the further ingredient(s).

## Revendications

1. Procédé de fabrication d'un mélange comprenant de la rotundone, comprenant les ou constitué des étapes suivantes consistant à:
a) faire réagir de l'/des huile(s) de bois, extrait(s) et/ou de la/des résine(s) naturelle(s), de préférence de l'huile de bois de gaïac, contenant du guajol et du bulnesol, contenant de préférence entre 15 et 45 % en poids de guajol et entre 25 et 55 % en poids de bulnesol, de manière particulièrement préférée contenant entre 20 et 35 % en poids de guajol et entre 30 et 45 % en poids de bulnesol, avec un ou plusieurs acide(s) organique(s),
b) séparer la fraction contenant du guajène du mélange obtenu à l'étape a), de préférence par distillation,
c) soumettre à l'oxydation la fraction séparée contenant du guajène issue de l'étape b), de préférence avec de l'air ambiant et/ou de l'oxygène pur comme agent oxydant,
d) en option: chauffer le mélange oxydé obtenu à l'étape c), de préférence chauffer sur une durée de 1 à 3 heures et/ou à 110 à 150 °C, de préférence à 115 à 125 °C,
e) séparer la fraction contenant de la rotundone du mélange obtenu à l'étape c) ou d), s'il est présent, de préférence par distillation, pour obtenir le mélange contenant de la rotundone.

2. Procédé selon la revendication 1, dans lequel le ou les acides organiques utilisés à l'étape a) est ou bien sont choisi(s) dans le groupe constitué par l'acide citrique, l'acide malique, l'acide tartrique et l'acide oxalique, de préférence dans lequel l'acide organique utilisé à l'étape a) est l'acide tartrique.

3. Procédé selon l'une quelconque des revendications précédentes, dans lequel à l'étape a) le rapport pondéral d'huile(s) de bois, d'extrait(s) et/ou de résine(s) naturelle(s) contenant du guajol et du bulnesol à l'acide organique ou bien à des acides organiques est compris entre 50 : 1 et 3 : 1, de préférence entre 20 : 1 et 2 : 1, de manière particulièrement préférée entre 12 : 1 et 8 : 1.

4. Procédé selon l'une quelconque des revendications précédentes, dans lequel la réaction à l'étape a) a lieu en présence d'un co-solvant ou de plusieurs co-solvants, de préférence en présence d'un polyéthylène glycol ou de plusieurs polyéthylène glycols, de manière particulièrement préférée en présence de PEG 600, et de préférence dans lequel le rapport pondéral d'huile(s) de bois, d'extrait(s) et/ou de résine(s) naturelle(s) contenant du guajol et du bulnesol audit un ou auxdits plusieurs co-solvant(s) est compris entre 4 : 1 et 1 : 2.

5. Procédé selon l'une quelconque des revendications précédentes, dans lequel la température de réaction de la réaction à l'étape a) est comprise entre 160 et 260 °C, de préférence entre 180 et 220 °C, et/ou la durée de réaction de la réaction à l'étape a) est comprise entre 1 et 20 h, de préférence entre 5 et 15 h, et/ou la réaction à l'étape a) a lieu à pression normale ou à pression négative, de préférence entre 400 et 900 mbar, de manière particulièrement préférée entre 500 et 700 mbar.

6. Procédé selon l'une quelconque des revendications précédentes, dans lequel à l'étape b) la séparation de la fraction contenant du guajène du mélange obtenu ou fourni à l'étape a) est réalisée par distillation, de préférence sous pression négative, de préférence entre 1,0 et 20,0 mbar et une température de fond comprise entre 90 et 175 °C, de manière particulièrement préférée entre 2,0 et 10,0 mbar et une température de fond comprise entre 105 et 160 °C, plus préférablement à 4,0 mbar et une température de fond de 130 °C.

7. Procédé selon l'une quelconque des revendications précédentes, dans lequel à l'étape c) l'oxydation de la fraction contenant du guajène issue de l'étape b) a lieu avec de l'air ambiant et/ou de l'oxygène pur comme agent oxydant et/ou à une température de 60 à 150 °C, de préférence 80 à 130 °C, de manière particulièrement préférée 90 à 110 °C, et/ou avec une quantité de gaz de l'agent oxydant de ≥ 10 à ≤ 1000 l/h, de préférence de ≥ 30 à ≤ 500 l/h, de manière particulièrement préférée de ≥ 40 à ≤ 100 l/h, par kg d'éduit et/ou sur une période de 10 à 60 h, de préférence de 15 à 30 h.

8. Procédé selon l'une quelconque des revendications précédentes, dans lequel l'indice de peroxyde du mélange obtenu à l'étape c) ou d), s'il est présent, est < 30 meq O/kg, de préférence < 20 meq O/kg.

9. Procédé selon l'une quelconque des revendications précédentes, dans lequel on ajoute au mélange à l'étape e), avant et/ou pendant la distillation, un ou plusieurs agents entraîneurs choisi(s) dans le groupe constitué de la triacétine et du citrate de triéthyle, dans un rapport pondéral du mélange obtenu à l'étape c) ou d), s'il est présent, à l'agent entraîneur de 4 :1 à 1 : 4, de préférence de 3 : 1 à 1 : 1,
et/ou
un ou plusieurs co-solvant(s) choisi(s) dans le groupe constitué par les polyéthylène glycols et le Palatinol Z, de préférence le PEG 400 et/ou le PEG 600, dans le rapport pondéral du mélange obtenu à l'étape c) ou d), s'il est présent, au(x) co-solvant(s) de 10 : 1 à 1 : 5, de préférence de 5 : 1 à 1 : 1.

10. Procédé selon l'une quelconque des revendications précédentes, dans lequel, à l'étape e), la séparation de la fraction contenant de la rotundone du mélange obtenu à l'étape c) ou d), s'il est présent, a lieu par distillation, de préférence sous pression négative, de préférence à 3 à 5 mbar.

11. Mélange contenant de la rotundone, comprenant ou constitué de rotundone, de bulnesen, de guajène, de guajol et de bulnesol ainsi qu'en option de citrate de triéthyle et/ou de triacétine.

12. Utilisation d'un mélange selon la revendication 11 pour générer, conférer ou modifier, de préférence intensifier, une ou plusieurs impressions gustatives et/ou olfactives, de préférence choisies dans le groupe constitué par les impressions d'agrumes, de fruits, de menthe, de muscade, de poivre, d'épices, d'herbes, de café, de chocolat, de noix, de nougat et de noix, de spiritueux conservés (par exemple les impressions de whisky, de rhum, de brandy), les impressions de vin et salées.

13. Préparation servant à la nutrition, à l'hygiène buccale ou au plaisir ou préparation cosmétique ou préparation pharmaceutique destinée à la prise orale ou produit semi-fini pour la fabrication d'une préparation servant à la nutrition, à l'hygiène buccale ou au plaisir ou préparation cosmétique ou préparation pharmaceutique destinée à la prise orale, comprenant un mélange selon la revendication 11.

14. Préparation ou produit semi-fini selon la revendication 13, comprenant en outre un ou plusieurs (autre(s)) agent(s) de sapidité et/ou substance(s) odorante(s), la quantité totale de mélange selon la revendication 11 étant suffisante pour modifier, de préférence intensifier une ou plusieurs impressions gustatives et/ou olfactives dudit ou desdits (autre(s)) agent(s) de sapidité et/ou substance(s) odorante(s).

15. Procédé de fabrication d'une préparation ou d'un produit semi-fini selon la revendication 13 ou 14, comprenant ou consistant en les étapes suivantes:
- fournir un mélange selon la revendication 11 et un ou plusieurs autres composants, dans lequel l'autre composant ou bien un autre composant ou plusieurs autres composants est/sont de préférence choisis dans le groupe constitué d'agents de sapidité et/ou de substances odorantes, et
- mélanger le mélange selon la revendication 11 avec ledit ou bien lesdits autre(s) composant(s).
